# EUROPEAN PATENT APPLICATION

(11) **EP 4 550 347 A1**
(43) Date of publication of application: **07.05.2025**
(21) Application number: 24206629.8
(22) Date of filing: 15.10.2024
(51) Int. Cl.: G16H 40/40

(54) **ELECTRONIC ACQUISITION OF TROUBLESHOOTING KNOWLEDGE FOR MEDICAL IMAGING SCANNERS**

(30) Priority: 02.11.2023 US 202318500691
(71) Applicant: GE Precision Healthcare LLC, Waukesha, WI 53188 (US)
(72) Inventor: MATSON CHAVARUKATTIL, Mathews, 560066 Bengaluru (IN); NARAYANA REDDY GARI, Rama Krishna Reddy, Waukesha, 53186 (US); MADHAVAN, Sridhar, Pewaukee, 53186-1118 (US)
(74) Representative: Fennell, Gareth Charles

(57) **Abstract**

Systems or techniques that facilitate electronic acquisition of troubleshooting knowledge for medical imaging scanners are provided. In various embodiments, a system can access a plain text service manual (e.g., 202) and a service complaint (e.g., 106) that are associated with a medical imaging scanner (e.g., 104). In various aspects, the system can identify, via named-entity recognition or natural language processing, a semantic hierarchy (e.g., 302) of the plain text service manual. In various instances, the system can record, via graphical user interface tracking, how a service technician (e.g., 602) that is troubleshooting the service complaint sequentially navigates through the semantic hierarchy, thereby yielding a troubleshooting trace (e.g., 504) that corresponds to the service complaint.

## Description

### TECHNICAL FIELD

The subject disclosure relates generally to medical imaging scanners, and more specifically to electronic acquisition of troubleshooting knowledge for medical imaging scanners.

### BACKGROUND

A medical imaging scanner can be deployed in the field. During deployment, the medical imaging scanner can malfunction. A service manual associated with the medical imaging scanner can contain information for solving the malfunction. However, the service manual can be voluminous, and locating such information within the service manual can thus be non-trivial. Existing techniques for locating such information require a user of the medical imaging scanner to manually sift through the service manual, assisted by nothing more than mere electronic keyword searches. Unfortunately, such existing techniques are unhelpful.

Accordingly, systems or techniques that can address one or more of these technical problems can be desirable.

### SUMMARY

The following presents a summary to provide a basic understanding of one or more embodiments. This summary is not intended to identify key or critical elements, or delineate any scope of the particular embodiments or any scope of the claims. Its sole purpose is to present concepts in a simplified form as a prelude to the more detailed description that is presented later. In one or more embodiments described herein, devices, systems, computer-implemented methods, apparatus or computer program products that facilitate electronic acquisition of troubleshooting knowledge for medical imaging scanners are described.

According to one or more embodiments, a system is provided. The system can comprise a non-transitory computer-readable memory that can store computer-executable components. The system can further comprise a processor that can be operably coupled to the non-transitory computer-readable memory and that can execute the computer-executable components stored in the non-transitory computer-readable memory. In various embodiments, the computer-executable components can comprise an access component that can access a plain text service manual and a service complaint that are associated with a medical imaging scanner. In various aspects, the computer-executable components can comprise a parsing component that can identify, via named-entity recognition or natural language processing, a semantic hierarchy of the plain text service manual. In various instances, the computer-executable components can comprise a trace component that can record, via graphical user interface tracking, how a service technician that is troubleshooting the service complaint sequentially navigates through the semantic hierarchy, thereby yielding a troubleshooting trace that corresponds to the service complaint. In various cases, the computer-executable components can comprise a model component that can train a deep learning neural network on the service complaint and on the troubleshooting trace, wherein the service complaint can be treated as a training input for the deep learning neural network, and wherein the troubleshooting trace can be treated as a ground-truth annotation corresponding to the service complaint.

According to one or more embodiments, a computer-implemented method is provided. In various embodiments, the computer-implemented method can comprise accessing, by a device operatively coupled to a processor, a plain text service manual and a service complaint that are associated with a medical imaging scanner. In various aspects, the computer-implemented method can comprise identifying, by the device and via named-entity recognition or natural language processing, a semantic hierarchy of the plain text service manual. In various instances, the computer-implemented method can comprise recording, by the device and via graphical user interface tracking, how a service technician that is troubleshooting the service complaint sequentially navigates through the semantic hierarchy, thereby yielding a troubleshooting trace that corresponds to the service complaint. In various cases, the computer-implemented method can comprise training, by the device, a deep learning neural network on the service complaint and on the troubleshooting trace, wherein the service complaint can be treated as a training input for the deep learning neural network, and wherein the troubleshooting trace can be treated as a ground-truth annotation corresponding to the service complaint.

According to one or more embodiments, a computer program product for facilitating electronic acquisition of troubleshooting knowledge is provided. In various embodiments, the computer program product can comprise a non-transitory computer-readable memory having program instructions embodied therewith. In various aspects, the program instructions can be executable by a processor to cause the processor to access one or more plain text documents associated with a machine. In various instances, the program instructions can be further executable to cause the processor to identify, via named-entity recognition or natural language processing, a semantic hierarchy of the one or more plain text documents. In various cases, the program instructions can be further executable to cause the processor to record, via graphical user interface tracking, how a service technician that is troubleshooting a service complaint associated with the machine sequentially navigates through the semantic hierarchy, thereby yielding a troubleshooting trace that corresponds to the service complaint. In various aspects, the program instructions can be further executable to cause the processor to train a deep learning neural network on the service complaint and on the troubleshooting trace, with the service complaint being a training input, and with the troubleshooting trace being a ground-truth annotation.

### DESCRIPTION OF THE DRAWINGS

FIG. 1 illustrates a block diagram of an example, non-limiting system that facilitates electronic acquisition of troubleshooting knowledge for medical imaging scanners in accordance with one or more embodiments described herein.
FIG. 2 illustrates a block diagram of an example, non-limiting system including a plain text service manual that facilitates electronic acquisition of troubleshooting knowledge for medical imaging scanners in accordance with one or more embodiments described herein.
FIG. 3 illustrates a block diagram of an example, non-limiting system including a semantic hierarchy that facilitates electronic acquisition of troubleshooting knowledge for medical imaging scanners in accordance with one or more embodiments described herein.
FIG. 4 illustrates an example, non-limiting block diagram showing how a semantic hierarchy can be generated in accordance with one or more embodiments described herein.
FIG. 5 illustrates a block diagram of an example, non-limiting system including a graphical user interface and a troubleshooting trace that facilitates electronic acquisition of troubleshooting knowledge for medical imaging scanners in accordance with one or more embodiments described herein.
FIG. 6 illustrates an example, non-limiting block diagram showing how a troubleshooting trace can be generated in accordance with one or more embodiments described herein.
FIG. 7 illustrates a block diagram of an example, non-limiting system including a deep learning neural network that facilitates electronic acquisition of troubleshooting knowledge for medical imaging scanners in accordance with one or more embodiments described herein.
FIG. 8 illustrates an example, non-limiting block diagram showing how a deep learning neural network can be trained on a troubleshooting trace in accordance with one or more embodiments described herein.
FIG. 9 illustrates a block diagram of an example, non-limiting system including an operational report that facilitates electronic acquisition of troubleshooting knowledge for medical imaging scanners in accordance with one or more embodiments described herein.
FIG. 10 illustrates a flow diagram of an example, non-limiting computer-implemented method that facilitates electronic acquisition of troubleshooting knowledge for medical imaging scanners in accordance with one or more embodiments described herein.
FIG. 11 illustrates a block diagram of an example, non-limiting operating environment in which one or more embodiments described herein can be facilitated.
FIG. 12 illustrates an example networking environment operable to execute various implementations described herein.

### DETAILED DESCRIPTION

The following detailed description is merely illustrative and is not intended to limit embodiments or application/uses of embodiments. Furthermore, there is no intention to be bound by any expressed or implied information presented in the preceding Background or Summary sections, or in the Detailed Description section.

One or more embodiments are now described with reference to the drawings, wherein like referenced numerals are used to refer to like elements throughout. In the following description, for purposes of explanation, numerous specific details are set forth in order to provide a more thorough understanding of the one or more embodiments. It is evident, however, in various cases, that the one or more embodiments can be practiced without these specific details.

A medical imaging scanner (e.g., a computed tomography (CT) scanner, a magnetic resonance imaging (MRI) scanner, an X-ray scanner, an ultrasound scanner, a positron emission tomography (PET) scanner) can be deployed in the field. Accordingly, the medical imaging scanner can capture or generate real-world medical scanned images (e.g., CT scanned images, MRI scanned images, X-ray scanned images, ultrasound scanned images, PET scanned images) of real-world medical patients (e.g., human, animal, or otherwise).

During deployment, the medical imaging scanner can malfunction. For instance, the medical imaging scanner can experience a hardware failure or a software failure that impedes or prevents the medical imaging scanner from properly capturing or generating medical scanned images.

A service manual (e.g., an electronic copy of a technical handbook) associated with the medical imaging scanner can contain information for solving the malfunction. However, the service manual can be voluminous (e.g., containing dozens or hundreds of pages of plain text). So, locating such information within the service manual can be non-trivial or otherwise difficult.

Existing techniques for locating such information require a user of the medical imaging scanner to manually sift through the service manual, assisted by nothing more than mere electronic keyword searches. In particular, such existing techniques involve the user selecting various keywords that they believe describe symptoms exhibited by the medical imaging scanner and electronically searching (e.g., via a FIND operation) within the service manual for those various keywords. Unfortunately, such existing techniques are unhelpful. Indeed, the user is prone to selecting keywords that are not utilized in the service manual or that otherwise do not accurately or properly describe symptoms of the malfunction. Furthermore, it is possible that many different types of malfunctions can manifest via common or shared symptoms. So, even if the user happens to select a keyword that accurately describes symptoms of the malfunction and that is actually recited in the service manual, electronically searching for such keyword can yield many potentially unrelated results for the user to review.

Accordingly, systems or techniques that can address one or more of these technical problems can be desirable.

Various embodiments described herein can address one or more of these technical problems. One or more embodiments described herein can include systems, computer-implemented methods, apparatus, or computer program products that can facilitate electronic acquisition of troubleshooting knowledge for medical imaging scanners. In particular, the inventors of various embodiments described herein realized that the service manual of the medical imaging scanner does not, by itself, represent the full or complete realm of information that is useful or helpful for solving the malfunction. Indeed, the present inventors realized that much rich information regarding how to solve the malfunction can be present, not just in the explicit words written in the service manual, but also in the sequential order in which a qualified service technician navigates through the service manual while troubleshooting the malfunction. That is, it is not just the written text of the service manual that can be considered as useful or informative for solving the malfunction; it is also the sequential order in which the qualified service technician reads through or consults such written text. Accordingly, the qualified service technician can troubleshoot the malfunction by referencing an electronically-displayed copy of the service manual, and various embodiments described herein can involve electronically tracking which specific sections of the service manual are visited in which specific order by the qualified service technician in solving the malfunction. Such tracking can be considered as capturing tacit, experiential knowledge of the qualified service technician that is helpful for solving the malfunction but that is not explicitly written in the service manual. When scaled across a myriad of malfunctions, such knowledge can be harnessed or aggregated by deep learning, such that an appropriate reading or navigation path through the service manual can be electronically predicted or inferred when given any particular malfunction of the medical imaging scanner.

Accordingly, various embodiments described herein can be considered as improving how the service manual can be leveraged to solve malfunctions experienced by the medical imaging scanner.

Various embodiments described herein can be considered as a computerized tool (e.g., any suitable combination of computer-executable hardware or computer-executable software) that can facilitate electronic acquisition of troubleshooting knowledge for medical imaging scanners. In various aspects, such computerized tool can comprise an access component, a parsing component, a trace component, or a model component.

In various embodiments, there can be a medical imaging scanner. In various aspects, the medical imaging scanner can be any suitable type of medical image-capture equipment or modality (e.g., a CT scanner, an MRI scanner, an X-ray scanner, an ultrasound scanner, or a PET scanner, among others). In various instances, the medical imaging scanner can be deployed in any suitable clinical or operational context (e.g., in a hospital, in a veterinary clinic, on an emergency vehicle). In various cases, the medical imaging scanner can comprise any suitable human-computer interface device (e.g., keyboard, keypad, touchscreen, voice command system).

In various embodiments, the medical imaging scanner can be associated with a service complaint. In various aspects, the service complaint can be any suitable electronic file that textually describes or otherwise indicates one or more hardware-based or software-based malfunction symptoms that are exhibited or demonstrated by the medical imaging scanner. In various instances, the service complaint can be electronically crafted, written, or generated by or at the behest of a user of the medical imaging scanner (e.g., the user can do so via the human-computer interface device of the medical imaging scanner).

In various embodiments, the access component of the computerized tool can electronically access the medical imaging scanner or the service complaint. For instance, the access component can electronically communicate with (e.g., transmit data to, receive data from) the medical imaging scanner. As another instance, the access component can electronically retrieve or obtain the service complaint from any suitable centralized or decentralized data structures (e.g., graph data structures, relational data structures, hybrid data structures), whether remote from or local to the access component. In any case, the access component can electronically access the medical imaging scanner or the service complaint, such that other components of the computerized tool can electronically interact with (e.g., read, write, edit, copy, manipulate) the medical imaging scanner or with the service complaint.

In various embodiments, the access component can further electronically access, from any suitable source, a plain text service manual associated with the medical imaging scanner. In various aspects, the plain text service manual can be an electronic document written in unstructured text (hence the term "plain text") that describes various technical or operational features, characteristics, or specifications regarding the medical imaging scanner. In various instances, the plain text service manual can be considered as an electronic factory handbook provided by a manufacturer of the medical imaging scanner and that textually explains how to operate, service, maintain, or repair the medical imaging scanner. In various cases, the plain text service manual can be voluminous (e.g., can be hundreds of pages long).

In various embodiments, the parsing component of the computerized tool can electronically generate a semantic hierarchy based on the plain text service manual. In various aspects, the parsing component can accomplish such generation by applying any suitable natural language processing techniques (e.g., named entity recognition) to the plain text service manual. In various instances, the semantic hierarchy can be a knowledge graph (e.g., comprising nodes and relations between nodes) that represents the semantic structure or organization of the plain text service manual (e.g., the semantic hierarchy can be considered as a type of document object model (DOM) tree of the plain text service manual). Accordingly, the semantic hierarchy can comprise a plurality of semantic nodes, various of which can be interrelated to each other via a plurality of semantic or organizational relations (e.g., a part-of relation, a nested-within relation). In various instances, the plurality of semantic nodes can respectively correspond to whatever semantic concepts (e.g., document title, document chapters, document keywords) that are textually recited or otherwise present in the plain text service manual. In various cases, each of the plurality of semantic nodes can be tagged with a respective location indicator specifying where (e.g., on what page, in what paragraph) that semantic node is located within the plain text service manual.

In various embodiments, the trace component of the computerized tool can electronically generate a troubleshooting trace, based on the service complaint and based on the semantic hierarchy. More specifically, the trace component can comprise or otherwise be electronically integrated with any suitable graphical user interface. In various aspects, the graphical user interface can comprise any suitable electronic display (e.g., computer screen, computer monitor) and any suitable human-computer interface device (e.g., keyboard, keypad, touchscreen). In various instances, the trace component can electronically render the plain text service manual on the graphical user interface. In various cases, a service technician who is qualified or certified to service the medical imaging scanner can attempt to solve the service complaint by troubleshooting (e.g., remotely or in person) the medical imaging scanner. In various aspects, the service technician can, during such troubleshooting, interact with the graphical user interface of the tracing component, so as to read through the plain text service manual in search of a solution to the service complaint. In various instances, the tracing component can electronically record which semantic nodes of the plain text service manual are visited by the service technician in which sequential order during such troubleshooting. In various cases, the tracing component can facilitate such recording by implementing any suitable graphical user interface tracking techniques (e.g., tracking which portions of the plain text service manual the service technician clicks on or scrolls to). In various aspects, such recorded data can be referred to as the troubleshooting trace. In other words, the troubleshooting trace can be considered as a directed path through the semantic hierarchy of the plain text service manual, which directed path indicates the sequential order in which the service technician navigates or reads through the plain text service manual in search for a solution to the service complaint. In still other words, the troubleshooting trace can be considered as indicating which specific portions of the plain text service manual were consulted in which specific order by the service technician so as to address the service complaint.

In various embodiments, the model component of the computerized tool can electronically train a deep learning neural network on the service complaint and on the troubleshooting trace.

More specifically, the model component can electronically store, maintain, control, or otherwise access the deep learning neural network. In various aspects, the deep learning neural network can exhibit any suitable internal architecture. For example, the deep learning neural network can include any suitable numbers of any suitable types of layers (e.g., input layer, one or more hidden layers, output layer, any of which can be convolutional layers, dense layers, non-linearity layers, pooling layers, batch normalization layers, or padding layers). As another example, the deep learning neural network can include any suitable numbers of neurons in various layers (e.g., different layers can have the same or different numbers of neurons as each other). As yet another example, the deep learning neural network can include any suitable activation functions (e.g., softmax, sigmoid, hyperbolic tangent, rectified linear unit) in various neurons (e.g., different neurons can have the same or different activation functions as each other). As still another example, the deep learning neural network can include any suitable interneuron connections or interlayer connections (e.g., forward connections, skip connections, recurrent connections).

In various aspects, the model component can train the deep learning neural network based on the service complaint and based on the troubleshooting trace. More specifically, the model component can treat the service complaint as a training input for the deep learning neural network, and the model component can treat the troubleshooting trace as a ground-truth annotation that is known or deemed to correspond to the service complaint.

In various cases, if the deep learning neural network has not yet undergone any training, the model component can randomly initialize the trainable internal parameters (e.g., convolutional kernels, weight matrices, bias vectors) of the deep learning neural network. In contrast, if the deep learning neural network has already undergone at least some training, the model component can refrain from re-initializing the trainable internal parameters of the deep learning neural network.

In various aspects, the model component can execute the deep learning neural network on the service complaint, thereby causing the deep learning neural network to produce some output. In particular, the model component can feed the service complaint to an input layer of the deep learning neural network, the service complaint can complete a forward pass through one or more hidden layers of the deep learning neural network, and such forward pass can cause an output layer of the deep learning neural network to compute the output based on activations provided by the one or more hidden layers.

Note that the format, size, or dimensionality of the output can be controlled or otherwise dictated by the number, arrangement, or sizes of the neurons or of other internal parameters (e.g., convolutional kernels) that are contained in or that otherwise make up the output layer of the deep learning neural network. So, the output can be forced to have any suitable or any desired format, size, or dimensionality, by adding, removing, or otherwise adjusting neurons or other internal parameters to, from, or within the output layer of the deep learning neural network. Accordingly, the output can be forced to have a same, comparable, or otherwise commensurate format, size, or dimensionality as the troubleshooting trace. So, the output can be considered as a predicted or inferred troubleshooting trace (e.g., a predicted or inferred reading sequence through the plain text service manual) that the deep learning neural network believes should correspond to the service complaint (e.g., believes would solve or address the service complaint). In contrast, the troubleshooting trace recorded by the trace component can be the correct or accurate reading sequence through the plain text service manual that is known or deemed to solve or address the service complaint (e.g., after all, the troubleshooting trace was derived from the service technician actually troubleshooting the service complaint). In various cases, if the deep learning neural network has so far undergone no or little training, the output can be highly inaccurate (e.g., can be very different from the troubleshooting trace).

In any case, the model component can compute an error or loss (e.g., mean absolute error (MAE), mean squared error (MSE), cross-entropy error) between the output and the troubleshooting trace. In various aspects, the model component can update the trainable internal parameters of the deep learning neural network by performing backpropagation (e.g., stochastic gradient descent) driven by the computed error or loss.

In various aspects, such training procedure can be repeated for any suitable number of service-complaint-and-troubleshooting-trace pairs. Such training can ultimately cause the trainable internal parameters of the deep learning neural network to become iteratively optimized for accurately inferring troubleshooting traces based on inputted service complaints. Note that the model component can implement any suitable training batch sizes, any suitable training termination criteria, or any suitable error, loss, or objective functions.

In various instances, after such training, the model component can electronically deploy the deep learning neural network as a third-party service. As a non-limiting example, a third-party who owns or operates an instantiation of the medical imaging scanner can provide or create a given service complaint, and the model component can execute the deep learning neural network on that given service complaint. Such execution can cause the deep learning neural network to produce a predicted troubleshooting trace, where such predicted troubleshooting trace can be considered as indicating a reading or navigation path through the plain text service manual that would (in the opinion of the deep learning neural network) solve or address the given service complaint. Accordingly, the third-party need not aimlessly sift through the plain text service manual. Instead, the tacit troubleshooting knowledge of the service technician can have been leveraged to train the deep learning neural network, and so the deep learning neural network can infer or predict which portions of the plain text service manual should be consulted in which sequential order so as to troubleshoot the given service complaint.

Various embodiments described herein can be employed to use hardware or software to solve problems that are highly technical in nature (e.g., to facilitate electronic acquisition of troubleshooting knowledge for medical imaging scanners), that are not abstract and that cannot be performed as a set of mental acts by a human. Further, some of the processes performed can be performed by a specialized computer (e.g., graphical user interfaces, text parsers, deep learning neural networks) for carrying out defined acts related to medical imaging scanners. For example, such defined acts can include: accessing, by a device operatively coupled to a processor, a plain text service manual and a service complaint that are associated with a medical imaging scanner; identifying, by the device and via named-entity recognition or natural language processing, a semantic hierarchy of the plain text service manual; and recording, by the device and via graphical user interface tracking, how a service technician that is troubleshooting the service complaint sequentially navigates through the semantic hierarchy, thereby yielding a troubleshooting trace that corresponds to the service complaint. Furthermore, in various instances, such defined acts can include: training, by the device, a deep learning neural network on the service complaint and on the troubleshooting trace, wherein the service complaint is treated as a training input for the deep learning neural network, and wherein the troubleshooting trace is treated as a ground-truth annotation corresponding to the service complaint.

Such defined acts are not performed manually by humans. Indeed, neither the human mind nor a human with pen and paper can electronically parse a plain text service manual of a medical imaging scanner (e.g., CT scanner, X-ray scanner) into a semantic hierarchy (e.g., a knowledge graph), electronically record (e.g., via click-tracking or scroll-tracking) which nodes of that semantic hierarchy are visually displayed on a graphical user interface at which times during troubleshooting of the medical imaging scanner, and electronically train a deep learning neural network on such recorded data. Indeed, medical imaging scanners, graphical user interfaces, and deep learning neural networks are inherently-computerized, hardware-and-software-based constructs that simply cannot be meaningfully implemented or trained in any way by the human mind without computers. A computerized tool that can electronically track a sequential reading or navigation order through a plain text service manual of a medical imaging scanner and utilize such sequential reading or navigation order to train a deep learning neural network to accurately predict sequential reading or navigation orders is likewise inherently-computerized and cannot be implemented in any sensible, practical, or reasonable way without computers.

Moreover, various embodiments described herein can integrate into a practical application various teachings relating to electronic acquisition of troubleshooting knowledge for medical imaging scanners. As described above, when a medical imaging scanner experiences a malfunction, existing techniques involve a user or owner of that medical imaging scanner aimlessly reading through a service manual of the medical imaging scanner in search for a solution to the malfunction. Such aimless reading is time-consuming and often ineffective (e.g., the user or owner is prone to sifting through irrelevant portions of the service manual, even when assisted with electronic keyword searches).

Various embodiments described herein can address one or more of these technical problems. In particular, the present inventors recognized that a distinct type of rich information that is helpful or useful for addressing malfunctions of the medical imaging scanner is not explicitly written within the service manual itself. Instead, as the present inventors realized, such distinct type of rich information is actually the sequential order in which an experienced, qualified, or certified service technician reads or navigates through the service manual when troubleshooting malfunctions. As described herein, various embodiments can involve recording such reading or navigation sequences via graphical user interface tracking (e.g., tracking which portions of the service manual the service technician clicks on, tracking which portions of the service manual the service technician scrolls to). In various aspects, such reading or navigation sequences can subsequently be leveraged to train a deep learning neural network. Accordingly, once trained, the deep learning neural network can respond to any given malfunction of the medical imaging scanner by predicting or inferring which specific portions of the service manual should be consulted in which specific order so as to solve the given malfunction. In this way, the user or owner of the medical imaging scanner need not aimlessly pore through the service manual and need not wait for an experienced, qualified, or certified service technician. Instead, the user or owner can troubleshoot the given malfunction themself, by relying upon the deep learning neural network to indicate which portions of the service manual should be consulted in which order (e.g., the user or owner need not waste time aimlessly reading through irrelevant portions of the service manual). For at least these reasons, various embodiments described herein be considered as an improved technique for automatically identifying relevant troubleshooting information in a service manual of a medical imaging scanner. Thus, various embodiments described herein certainly constitute a tangible and concrete technical improvement or technical advantage in the field of medical imaging scanners (e.g., existing techniques are not able to automatically and effectively identify which portions of a voluminous, plain text service manual of a medical imaging scanner are relevant for troubleshooting the medical imaging scanner). Accordingly, such embodiments clearly qualify as useful and practical applications of computers.

Furthermore, various embodiments described herein can control real-world tangible devices based on the disclosed teachings. For example, various embodiments described herein can electronically control real-world graphical user interfaces and can electronically train or execute real-world deep learning neural networks.

It should be appreciated that the herein figures and description provide non-limiting examples of various embodiments and are not necessarily drawn to scale.

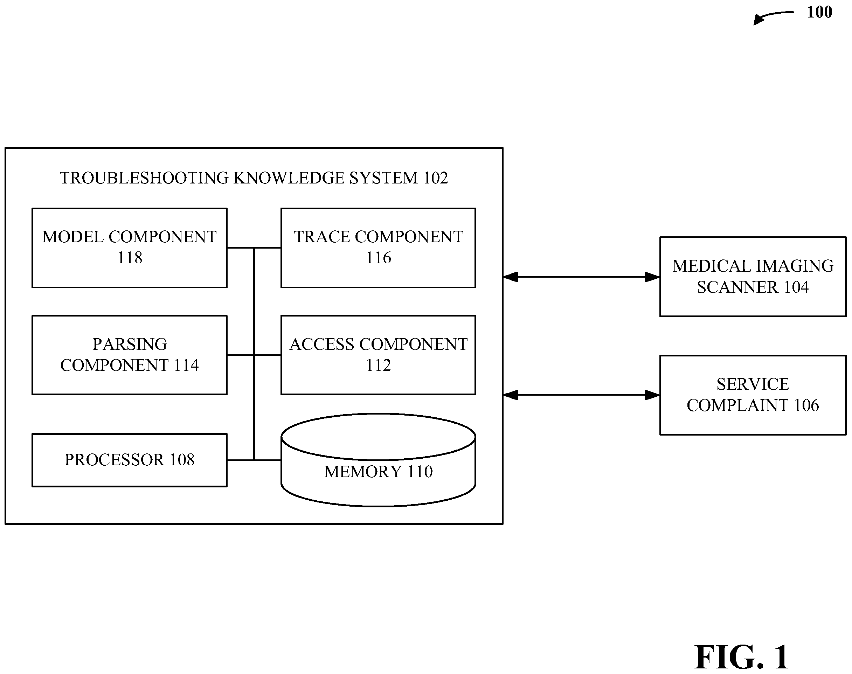

FIG. 1 illustrates a block diagram of an example, non-limiting system 100 that can facilitate electronic acquisition of troubleshooting knowledge for medical imaging scanners in accordance with one or more embodiments described herein. As shown, a troubleshooting knowledge system 102 can be electronically integrated, via any suitable wired or wireless electronic connections, with a medical imaging scanner 104 or with a service complaint 106.

In various embodiments, the medical imaging scanner 104 can be any suitable device, equipment, or modality for capturing or generating medical images. As a non-limiting example, the medical imaging scanner 104 can be a CT scanner that can capture or generate CT scanned pixel arrays or voxel arrays. As another non-limiting example, the medical imaging scanner 104 can be an MRI scanner that can capture or generate MRI scanned pixel arrays or voxel arrays. As even another non-limiting example, the medical imaging scanner 104 can be an X-ray scanner that can capture or generate X-ray scanned pixel arrays or voxel arrays. As yet another non-limiting example, the medical imaging scanner 104 can be an ultrasound scanner that can capture or generate ultrasound scanned pixel arrays or voxel arrays. As still another non-limiting example, the medical imaging scanner 104 can be a PET scanner that can capture or generate PET scanned pixel arrays or voxel arrays.

In various aspects, the medical imaging scanner 104 can be deployed, stationed, or otherwise implemented in any suitable clinical operational context. As a non-limiting example, the medical imaging scanner 104 can be deployed, stationed, or otherwise implemented within any suitable hospital or medical center. As another non-limiting example, the medical imaging scanner 104 can be deployed, stationed, or otherwise implemented within any suitable scientific or medical laboratory. As yet another non-limiting example, the medical imaging scanner 104 can be deployed, stationed, or otherwise implemented within any suitable veterinary center. As even another non-limiting example, the medical imaging scanner 104 can be deployed, stationed, or otherwise implemented within or onboard any suitable vehicle (e.g., ambulance, cruise ship, airplane).

In various instances, the medical imaging scanner 104 can comprise any suitable human-computer interface device by which a user or operator of the medical imaging scanner 104 can manually interact with or control the medical imaging scanner 104. As a non-limiting example, the medical imaging scanner 104 can comprise any suitable keyboard or keypad that can be pressed by the user or operator. As another non-limiting example, the medical imaging scanner 104 can comprise any suitable computer mouse that can be dragged or clicked by the user or operator. As even another non-limiting example, the medical imaging scanner 104 can comprise any suitable touchscreen that can be tactilely manipulated by the user or operator. As yet another non-limiting example, the medical imaging scanner 104 can comprise any suitable voice command system that can accept verbal instructions spoken by the user or operator.

In various cases, the service complaint 106 can be any suitable electronic data (e.g., one or more scalars, one or more vectors, one or more matrices, one or more tensors, one or more character strings, or any suitable combination thereof) that indicates, specifies, or otherwise conveys one or more malfunction symptoms that the medical imaging scanner 104 is afflicted with or is otherwise experiencing. As a non-limiting example, the service complaint 106 can be a plain text or structured text electronic file that describes such one or more malfunction symptoms. In various aspects, the user or operator of the medical imaging scanner 104 can generate or otherwise cause the service complaint 106 to be created, by interacting with the human-computer interface device of the medical imaging scanner 104 (e.g., by typing the service complaint 106 into a keyboard or touchscreen of the medical imaging scanner 104, by speaking the service complaint 106 into a voice command system of the medical imaging scanner 104). However, this is a mere non-limiting example. In other cases, the user or operator of the medical imaging scanner 104 can generate or otherwise cause the service complaint 106 to be created, by interacting with any other suitable computing device.

In various embodiments, the troubleshooting knowledge system 102 can comprise a processor 108 (e.g., computer processing unit, microprocessor) and a non-transitory computer-readable memory 110 that is operably or operatively or communicatively connected or coupled to the processor 108. The non-transitory computer-readable memory 110 can store computer-executable instructions which, upon execution by the processor 108, can cause the processor 108 or other components of the troubleshooting knowledge system 102 (e.g., access component 112, parsing component 114, trace component 116, model component 118) to perform one or more acts. In various embodiments, the non-transitory computer-readable memory 110 can store computer-executable components (e.g., access component 112, parsing component 114, trace component 116, model component 118), and the processor 108 can execute the computer-executable components.

In various embodiments, the troubleshooting knowledge system 102 can comprise an access component 112. In various aspects, the access component 112 can electronically access the medical imaging scanner 104 or the service complaint 106. As a non-limiting example, the access component 112 can electronically communicate in any suitable fashion with the medical imaging scanner 104. That is, the access component 112 can electronically transmit any suitable electronic data to the medical imaging scanner 104, and the medical imaging scanner 104 can likewise electronically transmit any suitable electronic data to the access component 112. As another non-limiting example, the access component 112 can electronically retrieve or otherwise electronically obtain the service complaint 106 from any suitable centralized or decentralized data structures (not shown) or from any suitable centralized or decentralized computing devices (not shown). Indeed, in some cases, the access component 112 can electronically receive the service complaint 106 from the medical imaging scanner 104. In any case, the access component 112 can electronically access the medical imaging scanner 104 or the service complaint 106, such that other components of the troubleshooting knowledge system 102 can electronically interact with the medical imaging scanner 104 or with the service complaint 106.

In various embodiments, the access component 112 can, as described herein, further access a plain text service manual corresponding to the medical imaging scanner 104.

In various embodiments, the troubleshooting knowledge system 102 can comprise a parsing component 114. In various aspects, as described herein, the parsing component 114 can generate a semantic hierarchy based on the plain text service manual.

In various embodiments, the troubleshooting knowledge system 102 can comprise a trace component 116. In various instances, as described herein, the trace component 116 can record a sequential order in which a service technician reads or navigates through the semantic hierarchy, and thus through the plain text service manual, while troubleshooting the service complaint. In some examples, such a recorded sequence can be referred to as a troubleshooting trace.

In various embodiments, the troubleshooting knowledge system 102 can comprise a model component 118. In various cases, as described herein, the model component 118 can train a deep learning neural network on the service complaint and on the troubleshooting trace.

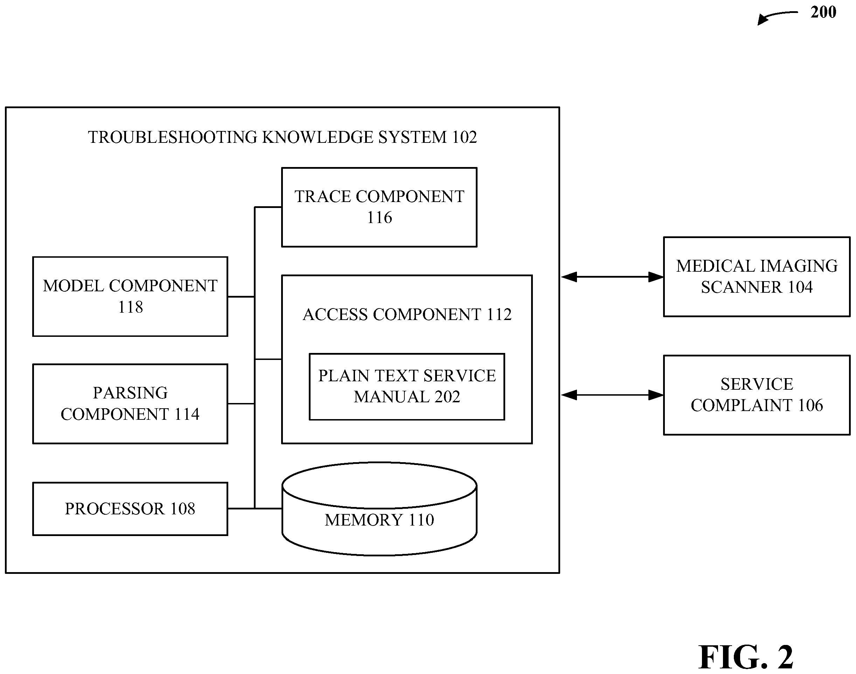

FIG. 2 illustrates a block diagram of an example, non-limiting system 200 including a plain text service manual that can facilitate electronic acquisition of troubleshooting knowledge for medical imaging scanners in accordance with one or more embodiments described herein. As shown, the system 200 can, in some cases, comprise the same components as the system 100, and can further comprise a plain text service manual 202.

In various embodiments, the access component 112 can electronically access the plain text service manual 202 from any suitable electronic source. As a non-limiting example, the access component 112 can electronically receive or retrieve the plain text service manual 202 from any suitable centralized or decentralized computing devices or databases (not shown).

In any case, the plain text service manual 202 can correspond to or otherwise be associated with the medical imaging scanner 104. In particular, the plain text service manual 202 can be any suitable electronic document that is written in plain, unstructured text (e.g., written in complete, human-readable sentences) and that can describe or explain how the medical imaging scanner 104 is to be technically operated, how the medical imaging scanner 104 is to be technically serviced, how the medical imaging scanner 104 is to be technically maintained, or how the medical imaging scanner 104 is to be technically repaired. Accordingly, the plain text service manual 202 can be considered as a manufacturer's handbook that conveys how to use and care for the medical imaging scanner 104. In various aspects, the plain text service manual 202 can exhibit any suitable electronic format. As a non-limiting example, the plain text service manual 202 can be in a portable document format (PDF). In various instances, the plain text service manual 202 can be exhibit any suitable size or length. As a non-limiting example, the plain text service manual 202 can be tens of electronic pages or hundreds of electronic pages long. In various cases, the plain text service manual 202 can be written in any suitable language, such as English, French, or Spanish.

In various aspects, the plain text service manual 202 can contain or otherwise describe information that would be helpful or useful for solving or addressing the service complaint 106. However, it can be initially unclear or unknown (e.g., by the user or operator of the medical imaging scanner 104) where such information is located within the plain text service manual 202. After all, it is possible that whatever words are recited in the service complaint 106 might not be recited verbatim in the plain text service manual 202, or might instead be recited at multiple, potentially irrelevant locations within the plain text service manual 202.

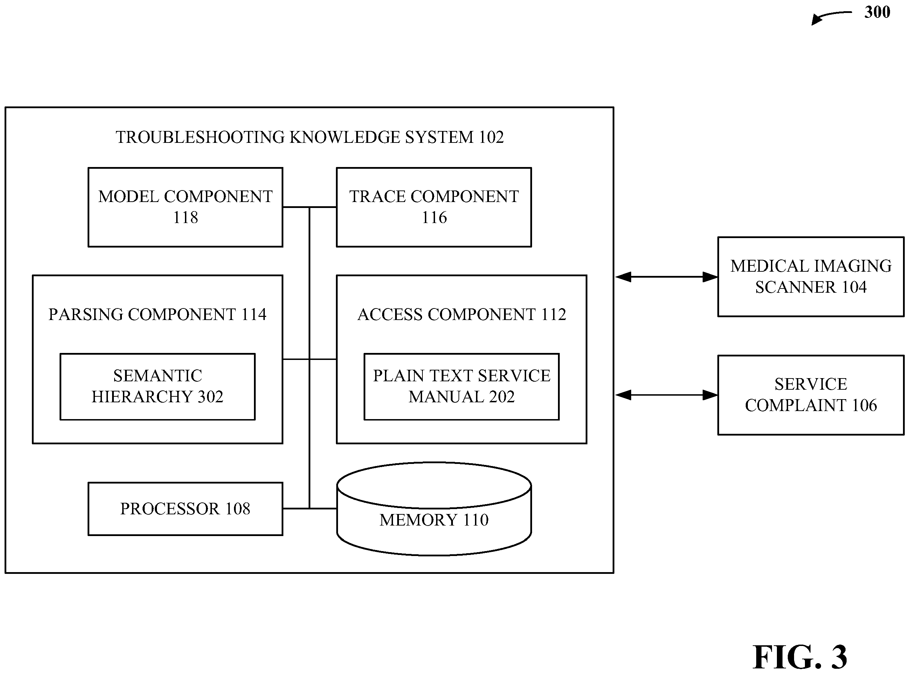

FIG. 3 illustrates a block diagram of an example, non-limiting system 300 including a semantic hierarchy that can facilitate electronic acquisition of troubleshooting knowledge for medical imaging scanners in accordance with one or more embodiments described herein. As shown, the system 300 can, in some cases, comprise the same components as the system 200, and can further comprise a semantic hierarchy 302.

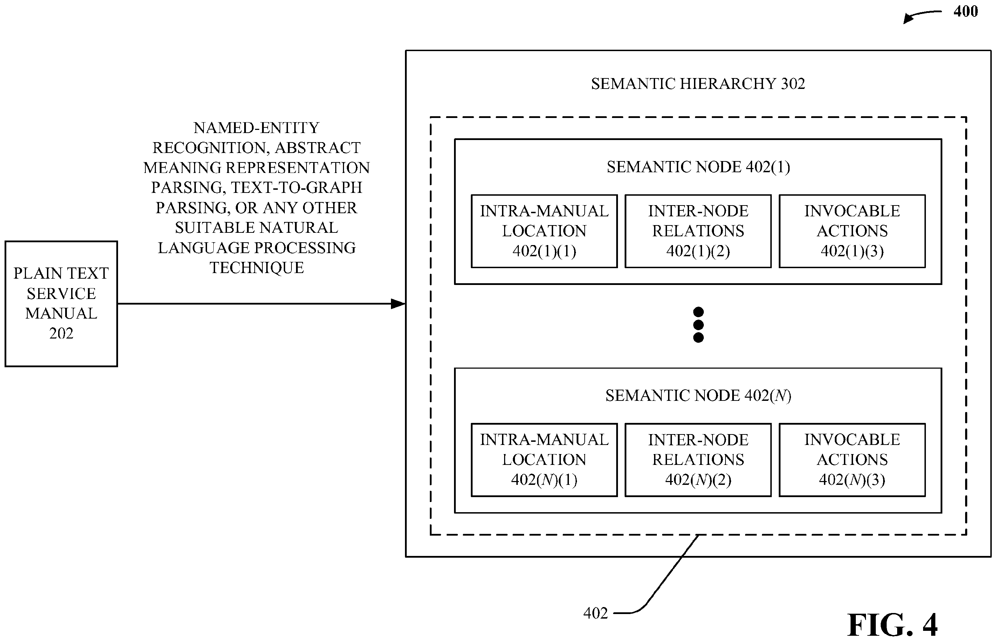

In various embodiments, the parsing component 114 can electronically generate the semantic hierarchy 302, based on the plain text service manual 202. In particular, the semantic hierarchy 302 can be considered as a knowledge graph that represents or otherwise conveys the semantic structure or semantic organization of the plain text service manual 202. Non-limiting aspects are described with respect to FIG. 4.

FIG. 4 illustrates an example, non-limiting block diagram 400 showing how the semantic hierarchy 302 can be generated in accordance with one or more embodiments described herein.

In various embodiments, as shown, the parsing component 114 can electronically generate the semantic hierarchy 302, by applying any suitable natural language processing techniques to the plain text service manual 202. As a non-limiting example, the parsing component 114 can generate the semantic hierarchy 302, by applying any suitable named-entity recognition technique to the plain text service manual 202. As another non-limiting example, the parsing component 114 can generate the semantic hierarchy 302, by applying any suitable abstract meaning representation parsing technique to the plain text service manual 202. As even another non-limiting example, the parsing component 114 can generate the semantic hierarchy 302, by applying any suitable text-to-graph parsing technique to the plain text service manual 202. As yet another non-limiting example, the parsing component 114 can generate the semantic hierarchy 302, by applying any suitable combination of any of the aforementioned natural language processing techniques to the plain text service manual 202.

In any case, the semantic hierarchy 302 can be considered as a knowledge graph that represents, conveys, indicates, or otherwise expresses the semantic structure or semantic organization of the plain text service manual 202. Accordingly, the semantic hierarchy 302 can comprise a plurality of semantic nodes 402. In various aspects, the plurality of semantic nodes 402 can comprise *n* nodes, for any suitable positive integer *n* > 1: a semantic node 402(1) to a semantic node 402(n). In various cases, each of the plurality of semantic nodes 402 can respectively correspond to any suitable semantic concept or semantic object that is explicitly present, written, or recited somewhere within the plain text service manual 202. For instance, the semantic node 402(1) can represent a first semantic concept or semantic object that is written or recited somewhere within the plain text service manual 202, whereas the semantic node 402(n) can represent an n-th semantic concept or semantic object that is written or recited somewhere within the plain text service manual 202. As some non-limiting examples, a semantic concept or semantic object can be: a title of the plain text service manual 202; a table of contents of the plain text service manual 202; an index or glossary of the plain text service manual 202; a chapter or section of the plain text service manual 202; a sub-chapter or subsection of the plain text service manual 202; a paragraph of the plain text service manual 202; an ordered list of the plain text service manual 202; an item within an ordered list of the plain text service manual 202; or a keyword of the plain text service manual 202 (e.g., a name of a particular hardware component of the medical imaging scanner 104; a name of a particular software component of the medical imaging scanner 104; a name of a particular hardware or software error or malfunction that can afflict the medical imaging scanner 104; a name of a particular hardware or software symptom that can be exhibited by the medical imaging scanner 104). Note that, given that the plain text service manual 202 can have a voluminous length, n can be quite large (e.g., can be in the thousands or even millions).

In various aspects, each of the plurality of semantic nodes 402 can be tagged or otherwise associated with a respective location within the plain text service manual 202, can have one or more respective relations with others of the plurality of semantic nodes 402, or can be associated with one or more invocable electronic actions.

As a non-limiting example, the semantic node 402(1) can be associated with an intra-manual location 402(1)(1), with one or more inter-node relations 402(1)(2), or with one or more invocable actions 402(1)(3).

In various instances, the intra-manual location 402(1)(1) can be any suitable electronic data (e.g., one or more scalars, one or more vectors, one or more matrices, one or more tensors, one or more character strings, or any suitable combination thereof) indicating where within the plain text service manual 202 whatever semantic concept or object that is represented by the semantic node 402(1) is located. In various cases, the intra-manual location 402(1)(1) can be specified at any suitable level of granularity (e.g., can indicate the electronic page number or the electronic line number at which the semantic concept or object represented by the semantic node 402(1) is located). Note that, in some aspects, the intra-manual location 402(1)(1) can indicate multiple locations, since whatever semantic concept or object that is represented by the semantic node 402(1) can possibly appear in multiple places within the plain text service manual 202 (e.g., can appear on multiple pages or in multiple lines).

In various aspects, the one or more inter-node relations 402(1)(2) can be any suitable electronic data (e.g., one or more scalars, one or more vectors, one or more matrices, one or more tensors, one or more character strings, or any suitable combination thereof) indicating which semantic relationships (e.g., directed or not directed) the semantic node 402(1) has with which other nodes of the plurality of a semantic nodes 402. Non-limiting examples of such semantic relationships can include: a subject-of relation; a part-of relation; a hyponym-of relation; a nested-within relation; a contains relation; a symptom-of relation; a caused-by relation; or a condition-precedent relation. Note that, in various cases, the one or more inter-node relations 402(1)(2) can be considered as being knowledge graph edges that are coupled to the semantic node 402(1).

In various aspects, the one or more invocable actions 402(1)(3) can be any suitable electronic data (e.g., one or more scalars, one or more vectors, one or more matrices, one or more tensors, one or more character strings, or any suitable combination thereof) indicating any suitable electronically-performable functions or operations that can be performed by, on, or otherwise with respect to the medical imaging scanner 104, in response to electronically invoking (e.g., clicking on) the semantic node 402(1) or whatever semantic concept or object is represented by the semantic node 402(1). For instance, suppose that the semantic node 402(1) represents the name of a particular hardware component of the medical imaging scanner 104. In such case, the one or more invocable actions 402(1)(3) can include a calibration or tuning protocol associated with that particular hardware component. So, invocation of (e.g., clicking on) the semantic node 402(1) (e.g., invoking or clicking on whatever semantic concept or object in the plain text service manual 202 that is represented by the semantic node 402(1)) can cause the medical imaging scanner 104 to automatically perform that calibration or tuning protocol on that particular hardware component. As another instance, suppose that the semantic node 402(1) instead represents the name of a particular software component of the medical imaging scanner 104. In such case, the one or more invocable actions 402(1)(3) can include pulling an operational log associated with that particular software component. So, invocation of (e.g., clicking on) the semantic node 402(1) (e.g., invoking or clicking on whatever semantic concept or object in the plain text service manual 202 that is represented by the semantic node 402(1)) can cause the medical imaging scanner 104 to automatically pull or generate that operational log for that particular software component. Note that, in some cases, the semantic node 402(1) can be associated with no invocable actions.

As another non-limiting example, the semantic node 402(n) can be associated with an intra-manual location 402(n)(1), with one or more inter-node relations 402(n)(2), or with one or more invocable actions 402(n)(3). As above, the intra-manual location 402(n)(1) can be any suitable electronic data indicating where within the plain text service manual 202 whatever semantic concept or object that is represented by the semantic node 402(n) is located. Also as above, the one or more inter-node relations 402(n)(2) can be any suitable electronic data indicating what semantic relations the semantic node 402(n) has with which others of the plurality of semantic nodes 402. Yet also as above, the one or more invocable actions 402(n)(3) can be any suitable electronically-performable functions or operations that can be performed by, on, or with respect to the medical imaging scanner 104, in response to invocation of the semantic node 402(n).

In any case, the semantic hierarchy 302 can be considered as a knowledge graph that represents or otherwise conveys the semantic architecture of the plain text service manual 202.

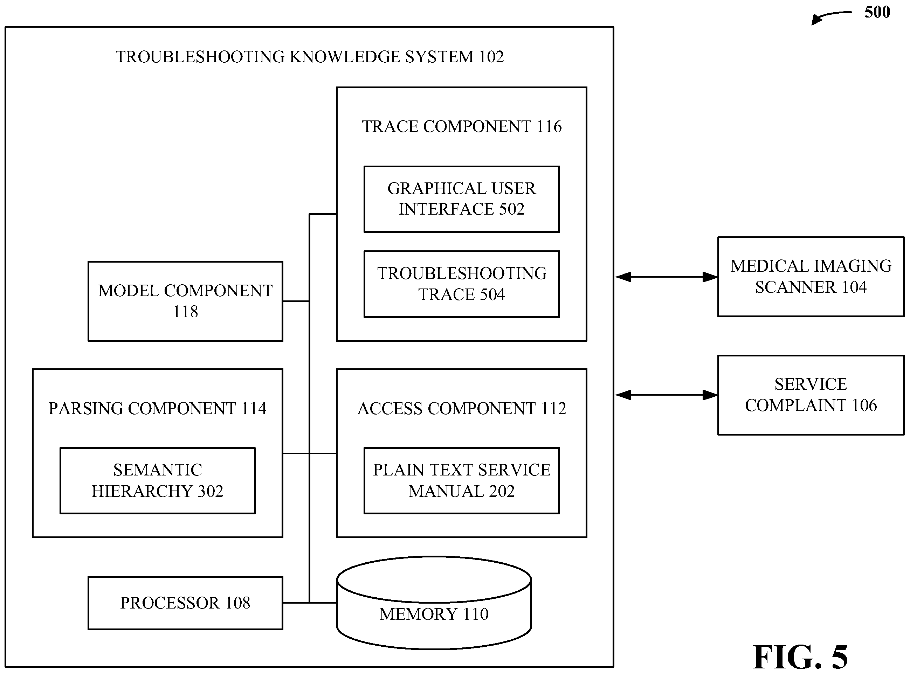

FIG. 5 illustrates a block diagram of an example, non-limiting system 500 including a graphical user interface and a troubleshooting trace that can facilitate electronic acquisition of troubleshooting knowledge for medical imaging scanners in accordance with one or more embodiments described herein. As shown, the system 500 can, in some cases, comprise the same components as the system 300, and can further comprise a graphical user interface 502 and a troubleshooting trace 504.

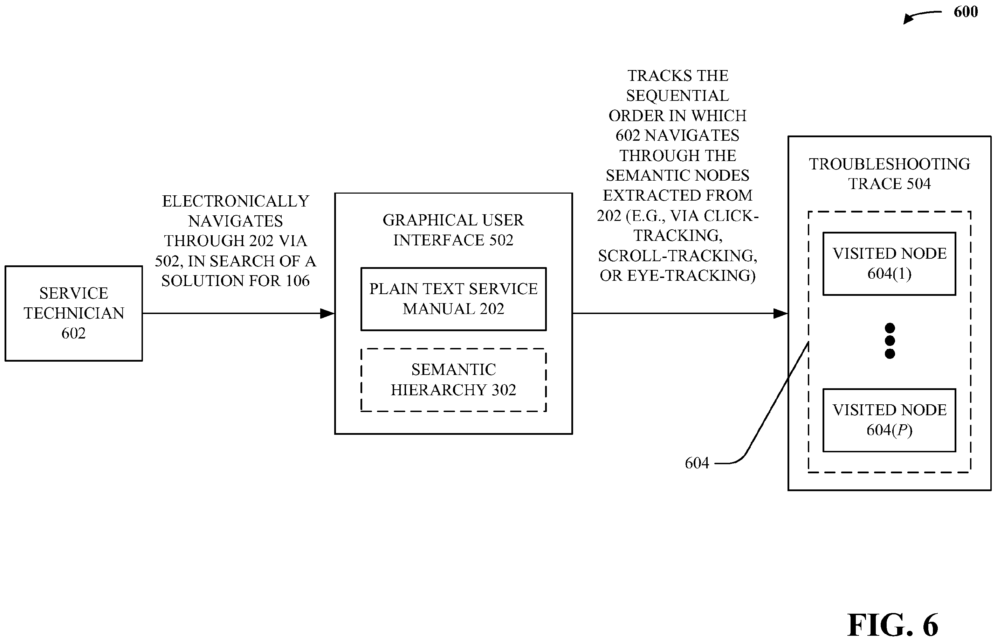

In various embodiments, the trace component 116 can electronically control or otherwise electronically access a graphical user interface 502. In various aspects, the graphical user interface 502 can comprise any suitable electronic display, such as a computer screen or a computer monitor. In various instances, the graphical user interface 502 can further comprise any suitable human-computer interface device, such as a keyboard, keypad, or voice command system. In some cases, the electronic display of the graphical user interface 502 can be an interactive touchscreen. In various aspects, the trace component 116 can visually render the plain text service manual 202 on the graphical user interface 502, such that a service technician who is responsible for troubleshooting the service complaint 106 can read through the plain text service manual 202 by viewing or interacting with the graphical user interface 502. In various instances, the graphical user interface 502 can electronically record or track the sequential order in which the service technician reads through the plain text service manual 202. In various cases, such sequential order can be considered as the troubleshooting trace 504. Non-limiting aspects are described with respect to FIG. 6.

FIG. 6 illustrates an example, non-limiting block diagram 600 showing how the troubleshooting trace 504 can be generated in accordance with one or more embodiments described herein.

In various embodiments, there can be a service technician 602. In various aspects, the service technician 602 can be any suitable licensed, certified, or otherwise qualified technician who is knowledgeable or experienced with respect to the technical operation, technical service, technical maintenance, or technical repair of the medical imaging scanner 104. Accordingly, the service technician 602 can troubleshoot or otherwise attempt to solve the service complaint 106.

In various instances, the graphical user interface 502 can visually render, depict, or otherwise illustrate the textual content of the plain text service manual 202 on its electronic display. In various cases, the service technician 602 can visually read or consult the plain text service manual 202, as displayed on the graphical user interface 502, while troubleshooting the service complaint 106. Note that, in some aspects, the service technician 602 can be physically present with or near the medical imaging scanner 104 (e.g., the service technician 602 can have made an in-person service trip to the medical imaging scanner 104). In such case, the graphical user interface 502 can be integrated with or otherwise be part of the medical imaging scanner 104. However, this is a mere non-limiting example. In other aspects, the service technician 602 can be physically present with or near the medical imaging scanner 104, and the graphical user interface 502 can be integrated into any other suitable computing device that is usable or viewable by the service technician 602 (e.g., can be part of a mobile device or tablet that accompanies the service technician 602 on service trips). In even other aspects, the service technician 602 can be remote from the medical imaging scanner 104, and the graphical user interface 502 can accordingly be integrated into any other suitable computing device that is usable or viewable by the service technician 602 (e.g., can be part of a desktop or computerized workstation of the service technician 602).

In any case, while troubleshooting the service complaint 106, the service technician 602 can interact with the graphical user interface 502, so as to electronically navigate through the plain text service manual 202. That is, the service technician 602 can read through or otherwise electronically visit various pages, various sections, or various paragraphs of the plain text service manual 202 in search of a solution for the service complaint 106. In various aspects, the trace component 116 can cause the graphical user interface 502 to electronically record or otherwise electronically track the sequential order in which the service technician 602 reads or navigates through the plain text service manual 202. In various instances, such recording or tracking can be facilitated by any suitable electronic tracing capabilities of the graphical user interface 502.

As a non-limiting example, the graphical user interface 502 can record such sequential order by leveraging scroll-tracking. That is, the graphical user interface 502 can record, know, or otherwise infer which portions of the plain text service manual 202 are visited or read by the service technician 602, and the sequential order in which they are visited or read, based on how the service technician 602 scrolls through the plain text service manual 202. For instance, suppose that the service technician 602 scrolls to a first page, section, paragraph, or word of the plain text service manual 202 and subsequently scrolls to a second page, section, paragraph, or word of the plain text service manual 202. In such case, it can be inferred that the service technician 602 read the second page, section, paragraph, or word after reading the first page, section, paragraph, or word while troubleshooting the service complaint 106. As another instance, suppose that the service technician 602 does not scroll to a third page, section, paragraph, or word of the plain text service manual 202. In such case, it can be inferred that the service technician 602 did not read the third page, section, paragraph, or word while troubleshooting the service complaint 106.

As another non-limiting example, the graphical user interface 502 can record such sequential order by leveraging click-tracking. That is, the graphical user interface 502 can record, know, or otherwise infer which portions of the plain text service manual 202 are visited or read by the service technician 602, and the sequential order in which they are visited or read, based on how the service technician 602 clicks through the plain text service manual 202. For instance, suppose that the service technician 602 clicks on a first page, section, paragraph, or word of the plain text service manual 202 and subsequently clicks on a second page, section, paragraph, or word of the plain text service manual 202. In such case, it can be inferred that the service technician 602 read the second page, section, paragraph, or word after reading the first page, section, paragraph, or word while troubleshooting the service complaint 106. As another instance, suppose that the service technician 602 does not click on a third page, section, paragraph, or word of the plain text service manual 202. In such case, it can be inferred that the service technician 602 did not read the third page, section, paragraph, or word while troubleshooting the service complaint 106.

As even another non-limiting example, the graphical user interface 502 can record such sequential order by leveraging eye-tracking. That is, the graphical user interface 502 can have a camera that monitors the eye movements or orientations of the service technician 602, the graphical user interface 502 can record, know, or otherwise infer which portions of the plain text service manual 202 are visited or read by the service technician 602, and the sequential order in which they are visited or read, based on how the eyes of the service technician 602 move with respect to the plain text service manual 202. For instance, suppose that the service technician 602 visually looks at a first page, section, paragraph, or word of the plain text service manual 202 for more than any suitable threshold amount of time and subsequently visually looks at a second page, section, paragraph, or word of the plain text service manual 202 for more than the threshold amount of time. In such case, it can be inferred that the service technician 602 read the second page, section, paragraph, or word after reading the first page, section, paragraph, or word while troubleshooting the service complaint 106. As another instance, suppose that the service technician 602 did not visually look at a third page, section, paragraph, or word of the plain text service manual 202 for more than the threshold amount of time. In such case, it can be inferred that the service technician 602 did not read the third page, section, paragraph, or word while troubleshooting the service complaint 106.

As yet another non-limiting example, the graphical user interface 502 can implement any suitable combination of scroll-tracking, click-tracking, or eye-tracking.

In any case, the graphical user interface 502 can electronically record or track the sequential order in which the service technician 602 reads or navigates through the plain text service manual 202 while troubleshooting the service complaint 106, and such sequential order can be considered or otherwise treated as the troubleshooting trace 504. Because the various locations within the plain text service manual 202 can respectively correspond to the plurality of semantic nodes 402 of the semantic hierarchy 302 (e.g., each semantic node can be tagged with one or more respective intra-manual location indicators), the troubleshooting trace 504 can thus be considered as a directed path through the semantic hierarchy 302.

In particular, the troubleshooting trace 504 can be any suitable electronic data (e.g., one or more scalars, one or more vectors, one or more matrices, one or more tensors, one or more character strings, or any suitable combination thereof) that can indicate a sequence of visited nodes 604. In various aspects, the sequence of visited nodes 604 can comprise p nodes, for any suitable positive integer p > 1: a visited node 604(1) to a visited node 604(p). In various aspects, the visited node 604(1) can be whichever of the plurality of semantic nodes 402 that the service technician 602 visited or navigated to (e.g., clicked on, scrolled to, looked at) initially or first while troubleshooting the service complaint 106. In contrast, the visited node 604(p) can be whichever of the plurality of semantic nodes 402 that the service technician 602 visited or navigated to (e.g., clicked on, scrolled to, looked at) last while troubleshooting the service complaint 106. In other words, the visited node 604(p) can be the last or final semantic node that the service technician 602 visited or navigated to before solving, addressing, or closing the service complaint 106.

Note that, in some cases, *p* can be less than *n,* since the service technician 602 can read or navigate through fewer than all of the plurality of semantic nodes 402 (e.g., can read or navigate through less than all of the plain text service manual 202) while troubleshooting the service complaint 106. However, note that, in other cases, *p* can be greater than *n,* since the service technician 602 can read or navigate to one or more of the plurality of semantic nodes 402 multiple times (e.g., can visit some portions of the plain text service manual 202 more than once) while troubleshooting the service complaint 106.

In some aspects, the troubleshooting trace 504 can be considered as a type of segmentation mask respectively corresponding to the nodes of the semantic hierarchy 302 (e.g., for each node, the troubleshooting trace 504 can indicate whether that node was visited and, if so, what sequential rank or place it had in the reading or navigation order employed by the service technician 602).

Note that, in some instances, the troubleshooting trace 504 can further indicate whether or not any semantic node that was visited by the service technician 602 was invoked. As a non-limiting example, and as mentioned above, the visited node 604(1) can be whichever of the plurality of semantic nodes 402 that the service technician 602 visited or navigated to first. In various cases, the troubleshooting trace 504 can indicate not just that such semantic node was visited or navigated to first during troubleshooting of the service complaint 106, but can also indicate whether or not the service technician 602 invoked or activated one or more of whatever electronically-invocable actions (if any) are associated with that semantic node.

In any case, the semantic hierarchy 302 can be a knowledge graph representing the semantic structure of the plain text service manual 202, and the troubleshooting trace 504 can represent or indicate a directed path through that knowledge graph, where such directed path indicates or otherwise represents the sequential order in which the service technician 602 reads or navigates through the plain text service manual 202 to solve or troubleshoot the service complaint 106.

In various embodiments, there can be any suitable number of distinct service complaints (e.g., multiple instances of 106) corresponding to the medical imaging scanner 104 (e.g., corresponding to any instantiation of the medical imaging scanner 104, where distinct instantiations can be deployed in distinct operational contexts). Accordingly, the trace component 116 can generate, as described above, a respective troubleshooting trace (e.g., multiple instances of 504) for each of those distinct service complaints. This can ultimately yield any suitable number of complaint-trace tuples or complaint-trace pairs, with each troubleshooting trace representing whatever reading or navigation sequence through the plain text service manual 202 solved or addressed a respective service complaint. Note that, in such cases, the parsing component 114 need not repeatedly or repetitively generate the semantic hierarchy 302.

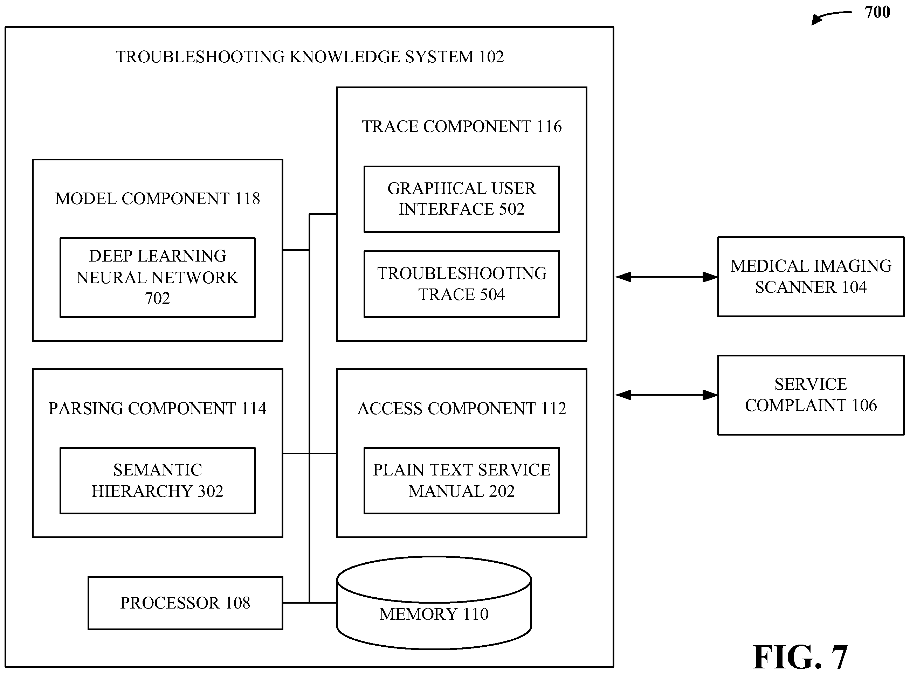

FIG. 7 illustrates a block diagram of an example, non-limiting system 700 including a deep learning neural network that can facilitate electronic acquisition of troubleshooting knowledge for medical imaging scanners in accordance with one or more embodiments described herein. As shown, the system 700 can, in some cases, comprise the same components as the system 500, and can further comprise a deep learning neural network 702.

In various embodiments, the model component 118 can electronically store, electronically maintain, electronically control, or otherwise electronically access the deep learning neural network 702. In various instances, the deep learning neural network 702 can have or otherwise exhibit any suitable deep learning internal architecture. For instance, the deep learning neural network 702 can have an input layer, one or more hidden layers, and an output layer. In various instances, any of such layers can be coupled together by any suitable interneuron connections or interlayer connections, such as forward connections, skip connections, or recurrent connections. Furthermore, in various cases, any of such layers can be any suitable types of neural network layers having any suitable learnable or trainable internal parameters. For example, any of such input layer, one or more hidden layers, or output layer can be convolutional layers, whose learnable or trainable parameters can be convolutional kernels. As another example, any of such input layer, one or more hidden layers, or output layer can be dense layers, whose learnable or trainable parameters can be weight matrices or bias values. As still another example, any of such input layer, one or more hidden layers, or output layer can be batch normalization layers, whose learnable or trainable parameters can be shift factors or scale factors. Further still, in various cases, any of such layers can be any suitable types of neural network layers having any suitable fixed or non-trainable internal parameters. For example, any of such input layer, one or more hidden layers, or output layer can be non-linearity layers, padding layers, pooling layers, or concatenation layers.

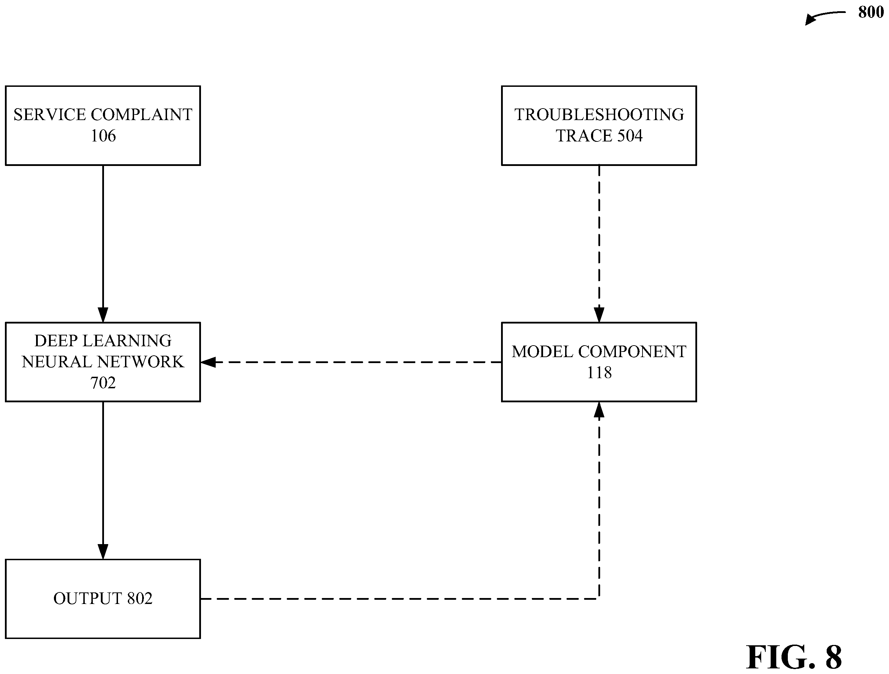

In various aspects, the model component 118 can electronically train the deep learning neural network 702 on the complaint-trace tuples acquired by the trace component 116. As a non-limiting example, the model component 118 can train the deep learning neural network 702 on the service complaint 106 and on the troubleshooting trace 504, where the model component 118 can treat the service complaint 106 as a training input, and where the model component 118 can treat the troubleshooting trace 504 as a ground-truth annotation corresponding to that training input. Non-limiting aspects are described with respect to FIG. 8.

FIG. 8 illustrates an example, non-limiting block diagram 800 showing how the deep learning neural network 702 can be trained on the troubleshooting trace 504 in accordance with one or more embodiments described herein.

Prior to beginning such training, the model component 118 can initialize in any suitable fashion (e.g., random initialization) the trainable internal parameters (e.g., weight matrices, bias vectors, convolutional kernels) of the deep learning neural network 702.

In various aspects, the model component 118 can electronically execute the deep learning neural network 702 on the service complaint 106, and such execution can cause the deep learning neural network 702 to produce an output 802. More specifically, as mentioned above, the service complaint 106 can be one or more scalars, one or more vectors, one or more matrices, one or more tensors, or one or more character strings. Accordingly, the model component 118 can feed the service complaint 106 (e.g., can feed whatever scalars, vectors, matrices, tensors, or character strings that define the service complaint 106) to an input layer of the deep learning neural network 702. In various instances, the service complaint 106 (e.g., whatever scalars, vectors, matrices, tensors, or character strings that define the service complaint 106) can complete a forward pass through one or more hidden layers of the deep learning neural network 702. In various cases, an output layer of the deep learning neural network 702 can compute the output 802, based on activation maps yielded by the one or more hidden layers.

Note that the format, size, or dimensionality of the output 802 can be dictated by the number, arrangement, sizes, or other characteristics of the neurons, convolutional kernels, or other internal parameters of the output layer (or of any other layers) of the deep learning neural network 702. Accordingly, the output 802 can be forced to have a format, size, or dimensionality that is the same as, comparable to, or otherwise commensurate with that of the troubleshooting trace 504. Thus, the output 802 can be considered as a predicted or inferred troubleshooting trace that the deep learning neural network 702 believes should correspond to the service complaint 106. In other words, the output 802 can be considered as a predicted or inferred reading or navigation sequence through the plain text service manual 202 that the deep learning neural network 702 believes would solve or address the service complaint 106. In contrast, the troubleshooting trace 504 can be considered as the ground-truth troubleshooting trace that is known or deemed to correspond to the service complaint 106. That is, the troubleshooting trace 504 can be the correct or accurate reading sequence through the plain text service manual 202 that is known or deemed to solve or address the service complaint 106. Note that, if the deep learning neural network 702 has so far undergone no or little training, then the output 802 can be highly inaccurate (e.g., can be very different from the troubleshooting trace 504).

In various aspects, the model component 118 can compute any suitable error or loss (e.g., MAE, MSE, cross-entropy) between the output 802 and the troubleshooting trace 504. In various instances, the model component 118 can update the trainable internal parameters of the deep learning neural network 702, by performing backpropagation (e.g., stochastic gradient descent) driven by the computed error or loss.

In various aspects, the above-described training procedure can be repeated for any suitable number of complaint-trace tuples (e.g., for each complaint-trace tuple acquired by the trace component 116). Such training can ultimately cause the trainable internal parameters of the deep learning neural network 702 to become iteratively optimized for accurately or correctly inferring troubleshooting traces (e.g., for accurately or correctly inferring reading or navigation sequences through the plain text service manual 202) in response to inputted service complaints. In various cases, the model component 118 can implement any suitable training termination criterion, any suitable training batch sizes, or any suitable error, loss, or objective functions when training the deep learning neural network 702.

In any case, the deep learning neural network 702 can thus be trained or configured to receive as input any service complaint associated with an instantiation of the medical imaging scanner 104, and to determine as output which specific portions of the plain text service manual 202 should be read in which specific order so as to solve or address that service complaint. Accordingly, the model component 118 can, in various embodiments, electronically deploy the deep learning neural network 702 as a third-party service. So, the deep learning neural network 702 can be considered as helping any third-party that owns or operates an instantiation of the medical imaging scanner 104 to more quickly or efficiently troubleshoot service complaints (e.g., without having to wait for a service technician, and without having to aimlessly sift through the plain text service manual 202). In other words, a computing device of a third-party can provide a given service complaint, the model component 118 can execute the deep learning neural network 702 on that given service complaint, thereby yielding a predicted troubleshooting trace, and the model component 118 can transmit that predicted troubleshooting trace back to the computing device of the third-party (e.g., the predicted troubleshooting trace can indicate the sequential order in which the third-party should read through the plain text service manual 202 so as to solve or address their given service complaint).

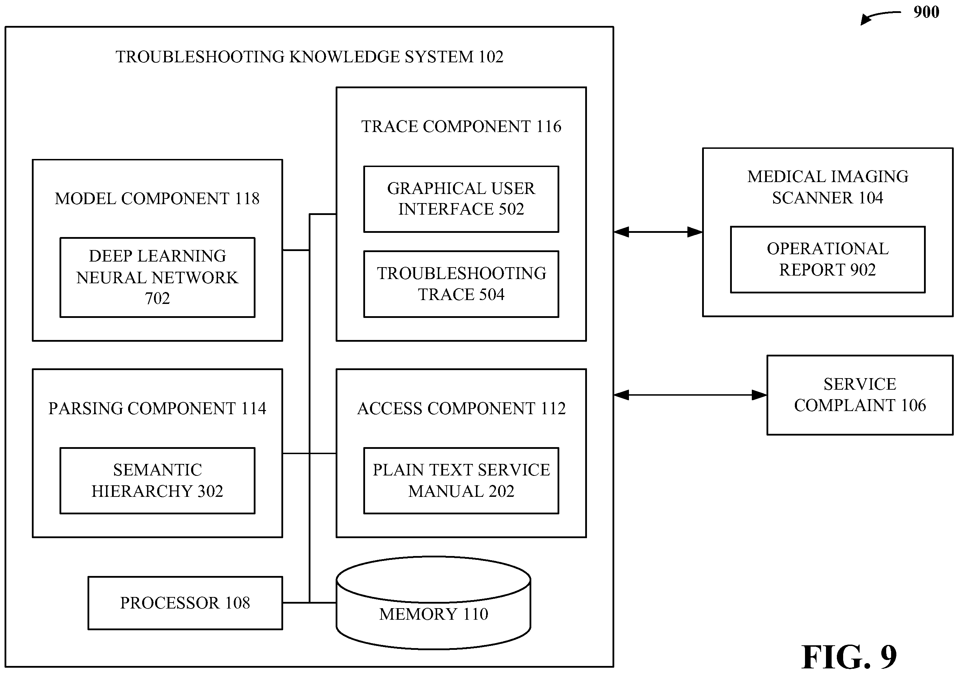

FIG. 9 illustrates a block diagram of an example, non-limiting system 900 including an operational report that can facilitate electronic acquisition of troubleshooting knowledge for medical imaging scanners in accordance with one or more embodiments described herein. As shown, the system 900 can, in some cases, comprise the same components as the system 700, and can further comprise an operational report 902.

In various embodiments, the access component 112 can electronically receive, retrieve, or otherwise access the operational report 902. In various aspects, the operational report 902 can be any suitable structured electronic textual document that is generatable by the medical imaging scanner 104 and that can represent, indicate, or otherwise convey current or present-time status information regarding the medical imaging scanner 104. As some non-limiting examples, the operational report 902 can be any suitable error log, calibration log, or tuning log that can be outputted by the medical imaging scanner 104.

In various instances, the parsing component 114 can generate the semantic hierarchy 302 based not only on the plain text service manual 202, but also based on the operational report 902. That is, the semantic hierarchy 302 can be the result obtained by applying any suitable natural language processing techniques (e.g., named entity recognition, abstract meaning representation parsing, or text-to-graph parsing) to both the plain text service manual 202 and to the operational report 902. Accordingly, the semantic hierarchy 302 can be a knowledge graph representing the semantic structure or organization of both the plain text service manual 202 and the operational report 902 (e.g., some of the plurality of semantic nodes 402 can represent semantic concepts or objects recited in the plain text service manual 202, whereas others of the plurality of semantic nodes 402 can represent semantic concepts or objects recited in the operational report 902). In such cases, the troubleshooting trace 504 can be considered as representing the sequential order in which the service technician 602 reads or navigates not only through the plain text service manual 202, but also through the operational report 902 (e.g., while troubleshooting the service complaint 106, the service technician 602 might jump back and forth between reading the plain text service manual 202 and reading the operational report 902). Furthermore, in such cases, the model component 118 can train the deep learning neural network 702 as described above, except that the training input to be fed to the deep learning neural network 702 can be both (e.g., the concatenation of) the service complaint 106 and the operational report 902. In such situations, the deep learning neural network 702 can be configured to receive as input a given service complaint and corresponding operational report, and to produce as output a troubleshooting trace that indicates the sequential order in which both the plain text service manual 202 and that corresponding operational report should be read so as to solve or address the given service complaint.

Note that the operational report 902 can, regardless of its specific substantive content, have an unchanging, standardized, or otherwise constant structure or format. So, the knowledge graph representation of each possible version of the operational report 902 can likewise be unchanging, standardized, or constant. Thus, just as above, there can be no need for the parsing component 114 to repeatedly or repetitively generate the semantic hierarchy 302.

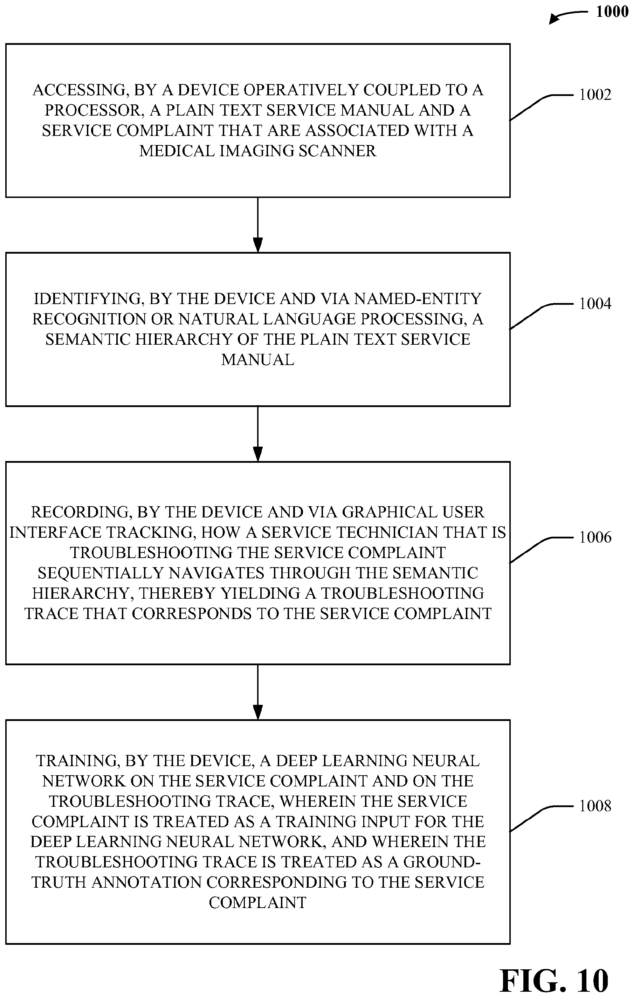

FIG. 10 illustrates a flow diagram of an example, non-limiting computer-implemented method 1000 that can facilitate electronic acquisition of troubleshooting knowledge for medical imaging scanners in accordance with one or more embodiments described herein. In various cases, the troubleshooting knowledge system 102 can facilitate the computer-implemented method 1000.

In various embodiments, act 1002 can include accessing, by a device (e.g., via 112) operatively coupled to a processor (e.g., 108), a plain text service manual (e.g., 202) and a service complaint (e.g., 106) that are associated with a medical imaging scanner (e.g., 104).

In various aspects, act 1004 can include identifying, by the device (e.g., via 114) and via named-entity recognition or natural language processing, a semantic hierarchy (e.g., 302) of the plain text service manual.

In various instances, act 1006 can include recording, by the device (e.g., via 116) and via graphical user interface tracking, how a service technician (e.g., 602) that is troubleshooting the service complaint sequentially navigates through the semantic hierarchy, thereby yielding a troubleshooting trace (e.g., 504) that corresponds to the service complaint.

In various cases, act 1008 can include training, by the device (e.g., via 118), a deep learning neural network (e.g., 702) on the service complaint and on the troubleshooting trace, wherein the service complaint can be treated as a training input for the deep learning neural network, and wherein the troubleshooting trace can be treated as a ground-truth annotation corresponding to the service complaint.

Although not explicitly shown in FIG. 10, the computer-implemented method 1000 can comprise: deploying, by the device (e.g., via 118), the deep learning neural network, after training, as a third-party service.

Although not explicitly shown in FIG. 10, the semantic hierarchy can be a knowledge graph representation of the plain text service manual, and the troubleshooting trace can be a directed path through the knowledge graph representation that indicates a reading sequence employed by the service technician through the plain text service manual to solve the service complaint.

Although not explicitly shown in FIG. 10, the computer-implemented method 1000 can comprise: accessing, by the device (e.g., via 112), an operational report (e.g., 902) generated by the medical imaging scanner, wherein the semantic hierarchy can be a knowledge graph representation of both the plain text service manual and the operational report, and wherein the troubleshooting trace can be a directed path through the knowledge graph representation that indicates a reading sequence employed by the service technician through both the plain text service manual and the operational report to solve the service complaint.

Although not explicitly shown in FIG. 10, the graphical user interface tracking can comprise click-tracking, scroll-tracking, or eye-movement-tracking.

Although not explicitly shown in FIG. 10, a node (e.g., 402(1)) of the semantic hierarchy can be based on a keyword located in the plain text service manual, the keyword can be associated with an electronic action (e.g., 402(1)(3)) performable by the medical imaging scanner, and clicking on or invoking the node can cause the medical imaging scanner to automatically perform the electronic action.

Thus far, various embodiments have been described with respect to plain text service manuals of medical imaging scanners. However, this is a mere non-limiting example. In various aspects, various embodiments described can be applied or otherwise extrapolated to any suitable textual documents of any suitable machines (e.g., even machines that are not medical imaging scanners).

For instance, various embodiments can include a computer program product for facilitating electronic acquisition of troubleshooting knowledge. In various aspects, the computer program product can comprise a non-transitory computer-readable memory (e.g., 110) having program instructions embodied therewith. In various instances, the program instructions can be executable by a processor (e.g., 108) to cause the processor to: access one or more plain text documents (e.g., 202, 902) associated with a machine (e.g., 104); identify, via named-entity recognition or natural language processing, a semantic hierarchy (e.g., 302) of the one or more plain text documents; record, via graphical user interface tracking, how a service technician (e.g., 602) that is troubleshooting a service complaint (e.g., 106) associated with the machine sequentially navigates through the semantic hierarchy, thereby yielding a troubleshooting trace (e.g., 504) that corresponds to the service complaint; and train a deep learning neural network (e.g., 702) on the service complaint and on the troubleshooting trace, with the service complaint being a training input, and with the troubleshooting trace being a ground-truth annotation. In various cases, the semantic hierarchy can be a knowledge graph representation of the one or more plain text documents, and the troubleshooting trace can be a directed path through the knowledge graph representation that indicates a reading sequence employed by the service technician through the one or more plain text documents to solve the service complaint. In various aspects, the one or more plain text documents can comprise a service manual (e.g., 202) of the machine or an operational report (e.g., 902) produced by the machine. In various instances, the graphical user interface tracking can comprise click-tracking, scroll-tracking, or eye-movement-tracking. In various cases, the program instructions can be further executable to cause the processor to: deploy the deep learning neural network, after training, as a third-party service. In various aspects, such deployment can comprise: receiving, from a third-party computing device, a third-party service complaint associated with the machine; executing the deep learning neural network on the third-party service complaint, thereby yielding an inferred troubleshooting trace, wherein the inferred troubleshooting trace can represent a sequential reading path through the one or more plain text documents which is predicted to solve or address the third-party service complaint; and transmitting, to the third-party computing device, the inferred troubleshooting trace.

In various instances, machine learning algorithms or models can be implemented in any suitable way to facilitate any suitable aspects described herein. To facilitate some of the above-described machine learning aspects of various embodiments, consider the following discussion of artificial intelligence (AI). Various embodiments described herein can employ artificial intelligence to facilitate automating one or more features or functionalities. The components can employ various AI-based schemes for carrying out various embodiments/examples disclosed herein. In order to provide for or aid in the numerous determinations (e.g., determine, ascertain, infer, calculate, predict, prognose, estimate, derive, forecast, detect, compute) described herein, components described herein can examine the entirety or a subset of the data to which it is granted access and can provide for reasoning about or determine states of the system or environment from a set of observations as captured via events or data. Determinations can be employed to identify a specific context or action, or can generate a probability distribution over states, for example. The determinations can be probabilistic; that is, the computation of a probability distribution over states of interest based on a consideration of data and events. Determinations can also refer to techniques employed for composing higher-level events from a set of events or data.

Such determinations can result in the construction of new events or actions from a set of observed events or stored event data, whether or not the events are correlated in close temporal proximity, and whether the events and data come from one or several event and data sources. Components disclosed herein can employ various classification (explicitly trained (e.g., via training data) as well as implicitly trained (e.g., via observing behavior, preferences, historical information, receiving extrinsic information, and so on)) schemes or systems (e.g., support vector machines, neural networks, expert systems, Bayesian belief networks, fuzzy logic, data fusion engines, and so on) in connection with performing automatic or determined action in connection with the claimed subject matter. Thus, classification schemes or systems can be used to automatically learn and perform a number of functions, actions, or determinations.

A classifier can map an input attribute vector, z = (zi, z₂, z₃, z₄, z*ₙ*), to a confidence that the input belongs to a class, as by f(z) = *confidence*(*class*). Such classification can employ a probabilistic or statistical-based analysis (e.g., factoring into the analysis utilities and costs) to determinate an action to be automatically performed. A support vector machine (SVM) can be an example of a classifier that can be employed. The SVM operates by finding a hyper-surface in the space of possible inputs, where the hyper-surface attempts to split the triggering criteria from the non-triggering events. Intuitively, this makes the classification correct for testing data that is near, but not identical to training data. Other directed and undirected model classification approaches include, e.g., naive Bayes, Bayesian networks, decision trees, neural networks, fuzzy logic models, or probabilistic classification models providing different patterns of independence, any of which can be employed. Classification as used herein also is inclusive of statistical regression that is utilized to develop models of priority.

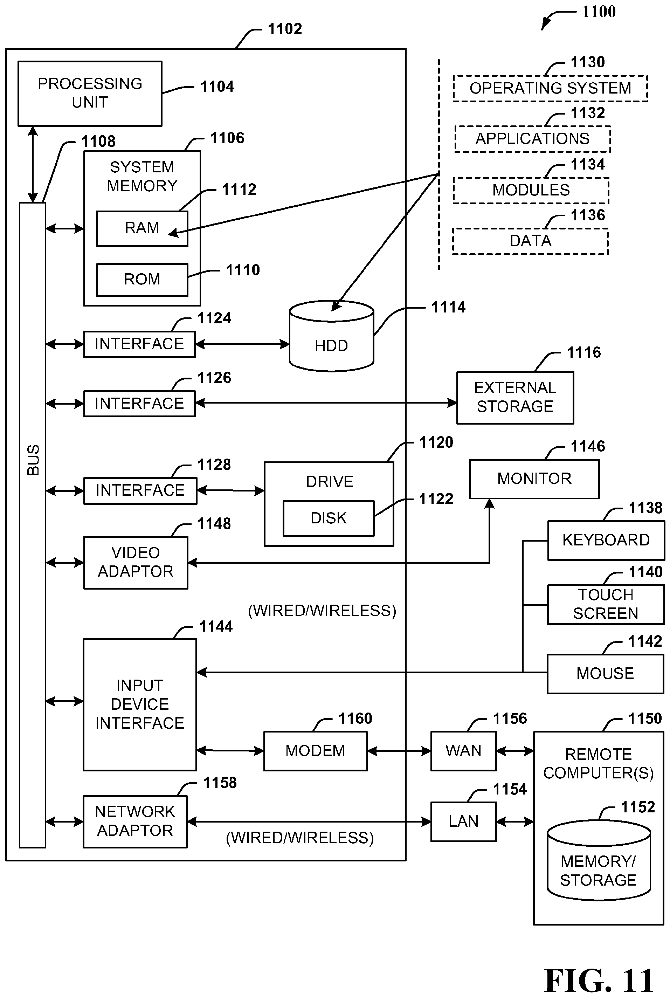

In order to provide additional context for various embodiments described herein, FIG. 11 and the following discussion are intended to provide a brief, general description of a suitable computing environment 1100 in which the various embodiments of the embodiment described herein can be implemented. While the embodiments have been described above in the general context of computer-executable instructions that can run on one or more computers, those skilled in the art will recognize that the embodiments can be also implemented in combination with other program modules or as a combination of hardware and software.

Generally, program modules include routines, programs, components, data structures, etc., that perform particular tasks or implement particular abstract data types. Moreover, those skilled in the art will appreciate that the inventive methods can be practiced with other computer system configurations, including single-processor or multi-processor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments of the embodiments herein can be also practiced in distributed computing environments where certain tasks are performed by remote processing devices that are linked through a communications network. In a distributed computing environment, program modules can be located in both local and remote memory storage devices.

Computing devices typically include a variety of media, which can include computer-readable storage media, machine-readable storage media, or communications media, which two terms are used herein differently from one another as follows. Computer-readable storage media or machine-readable storage media can be any available storage media that can be accessed by the computer and includes both volatile and nonvolatile media, removable and non-removable media. By way of example, and not limitation, computer-readable storage media or machine-readable storage media can be implemented in connection with any method or technology for storage of information such as computer-readable or machine-readable instructions, program modules, structured data or unstructured data.

Computer-readable storage media can include, but are not limited to, random access memory (RAM), read only memory (ROM), electrically erasable programmable read only memory (EEPROM), flash memory or other memory technology, compact disk read only memory (CD-ROM), digital versatile disk (DVD), Blu-ray disc (BD) or other optical disk storage, magnetic cassettes, magnetic tape, magnetic disk storage or other magnetic storage devices, solid state drives or other solid state storage devices, or other tangible or non-transitory media which can be used to store desired information. In this regard, the terms "tangible" or "non-transitory" herein as applied to storage, memory or computer-readable media, are to be understood to exclude only propagating transitory signals per se as modifiers and do not relinquish rights to all standard storage, memory or computer-readable media that are not only propagating transitory signals per se.

Computer-readable storage media can be accessed by one or more local or remote computing devices, e.g., via access requests, queries or other data retrieval protocols, for a variety of operations with respect to the information stored by the medium.

Communications media typically embody computer-readable instructions, data structures, program modules or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, RF, infrared and other wireless media.

With reference again to FIG. 11, the example environment 1100 for implementing various embodiments of the aspects described herein includes a computer 1102, the computer 1102 including a processing unit 1104, a system memory 1106 and a system bus 1108. The system bus 1108 couples system components including, but not limited to, the system memory 1106 to the processing unit 1104. The processing unit 1104 can be any of various commercially available processors. Dual microprocessors and other multi-processor architectures can also be employed as the processing unit 1104.

The system bus 1108 can be any of several types of bus structure that can further interconnect to a memory bus (with or without a memory controller), a peripheral bus, and a local bus using any of a variety of commercially available bus architectures. The system memory 1106 includes ROM 1110 and RAM 1112. A basic input/output system (BIOS) can be stored in a non-volatile memory such as ROM, erasable programmable read only memory (EPROM), EEPROM, which BIOS contains the basic routines that help to transfer information between elements within the computer 1102, such as during startup. The RAM 1112 can also include a high-speed RAM such as static RAM for caching data.

The computer 1102 further includes an internal hard disk drive (HDD) 1114 (e.g., EIDE, SATA), one or more external storage devices 1116 (e.g., a magnetic floppy disk drive (FDD) 1116, a memory stick or flash drive reader, a memory card reader, etc.) and a drive 1120, e.g., such as a solid state drive, an optical disk drive, which can read or write from a disk 1122, such as a CD-ROM disc, a DVD, a BD, etc. Alternatively, where a solid state drive is involved, disk 1122 would not be included, unless separate. While the internal HDD 1114 is illustrated as located within the computer 1102, the internal HDD 1114 can also be configured for external use in a suitable chassis (not shown). Additionally, while not shown in environment 1100, a solid state drive (SSD) could be used in addition to, or in place of, an HDD 1114. The HDD 1114, external storage device(s) 1116 and drive 1120 can be connected to the system bus 1108 by an HDD interface 1124, an external storage interface 1126 and a drive interface 1128, respectively. The interface 1124 for external drive implementations can include at least one or both of Universal Serial Bus (USB) and Institute of Electrical and Electronics Engineers (IEEE) 1394 interface technologies. Other external drive connection technologies are within contemplation of the embodiments described herein.

The drives and their associated computer-readable storage media provide nonvolatile storage of data, data structures, computer-executable instructions, and so forth. For the computer 1102, the drives and storage media accommodate the storage of any data in a suitable digital format. Although the description of computer-readable storage media above refers to respective types of storage devices, it should be appreciated by those skilled in the art that other types of storage media which are readable by a computer, whether presently existing or developed in the future, could also be used in the example operating environment, and further, that any such storage media can contain computer-executable instructions for performing the methods described herein.

A number of program modules can be stored in the drives and RAM 1112, including an operating system 1130, one or more application programs 1132, other program modules 1134 and program data 1136. All or portions of the operating system, applications, modules, or data can also be cached in the RAM 1112. The systems and methods described herein can be implemented utilizing various commercially available operating systems or combinations of operating systems.

Computer 1102 can optionally comprise emulation technologies. For example, a hypervisor (not shown) or other intermediary can emulate a hardware environment for operating system 1130, and the emulated hardware can optionally be different from the hardware illustrated in FIG. 11. In such an embodiment, operating system 1130 can comprise one virtual machine (VM) of multiple VMs hosted at computer 1102. Furthermore, operating system 1130 can provide runtime environments, such as the Java runtime environment or the .NET framework, for applications 1132. Runtime environments are consistent execution environments that allow applications 1132 to run on any operating system that includes the runtime environment. Similarly, operating system 1130 can support containers, and applications 1132 can be in the form of containers, which are lightweight, standalone, executable packages of software that include, e.g., code, runtime, system tools, system libraries and settings for an application.

Further, computer 1102 can be enable with a security module, such as a trusted processing module (TPM). For instance with a TPM, boot components hash next in time boot components, and wait for a match of results to secured values, before loading a next boot component. This process can take place at any layer in the code execution stack of computer 1102, e.g., applied at the application execution level or at the operating system (OS) kernel level, thereby enabling security at any level of code execution.

A user can enter commands and information into the computer 1102 through one or more wired/wireless input devices, e.g., a keyboard 1138, a touch screen 1140, and a pointing device, such as a mouse 1142. Other input devices (not shown) can include a microphone, an infrared (IR) remote control, a radio frequency (RF) remote control, or other remote control, a joystick, a virtual reality controller or virtual reality headset, a game pad, a stylus pen, an image input device, e.g., camera(s), a gesture sensor input device, a vision movement sensor input device, an emotion or facial detection device, a biometric input device, e.g., fingerprint or iris scanner, or the like. These and other input devices are often connected to the processing unit 1104 through an input device interface 1144 that can be coupled to the system bus 1108, but can be connected by other interfaces, such as a parallel port, an IEEE 1394 serial port, a game port, a USB port, an IR interface, a BLUETOOTH^{®} interface, etc.

A monitor 1146 or other type of display device can be also connected to the system bus 1108 via an interface, such as a video adapter 1148. In addition to the monitor 1146, a computer typically includes other peripheral output devices (not shown), such as speakers, printers, etc.

The computer 1102 can operate in a networked environment using logical connections via wired or wireless communications to one or more remote computers, such as a remote computer(s) 1150. The remote computer(s) 1150 can be a workstation, a server computer, a router, a personal computer, portable computer, microprocessor-based entertainment appliance, a peer device or other common network node, and typically includes many or all of the elements described relative to the computer 1102, although, for purposes of brevity, only a memory/storage device 1152 is illustrated. The logical connections depicted include wired/wireless connectivity to a local area network (LAN) 1154 or larger networks, e.g., a wide area network (WAN) 1156. Such LAN and WAN networking environments are commonplace in offices and companies, and facilitate enterprise-wide computer networks, such as intranets, all of which can connect to a global communications network, e.g., the Internet.

When used in a LAN networking environment, the computer 1102 can be connected to the local network 1154 through a wired or wireless communication network interface or adapter 1158. The adapter 1158 can facilitate wired or wireless communication to the LAN 1154, which can also include a wireless access point (AP) disposed thereon for communicating with the adapter 1158 in a wireless mode.

When used in a WAN networking environment, the computer 1102 can include a modem 1160 or can be connected to a communications server on the WAN 1156 via other means for establishing communications over the WAN 1156, such as by way of the Internet. The modem 1160, which can be internal or external and a wired or wireless device, can be connected to the system bus 1108 via the input device interface 1144. In a networked environment, program modules depicted relative to the computer 1102 or portions thereof, can be stored in the remote memory/storage device 1152. It will be appreciated that the network connections shown are example and other means of establishing a communications link between the computers can be used.

When used in either a LAN or WAN networking environment, the computer 1102 can access cloud storage systems or other network-based storage systems in addition to, or in place of, external storage devices 1116 as described above, such as but not limited to a network virtual machine providing one or more aspects of storage or processing of information. Generally, a connection between the computer 1102 and a cloud storage system can be established over a LAN 1154 or WAN 1156 e.g., by the adapter 1158 or modem 1160, respectively. Upon connecting the computer 1102 to an associated cloud storage system, the external storage interface 1126 can, with the aid of the adapter 1158 or modem 1160, manage storage provided by the cloud storage system as it would other types of external storage. For instance, the external storage interface 1126 can be configured to provide access to cloud storage sources as if those sources were physically connected to the computer 1102.

The computer 1102 can be operable to communicate with any wireless devices or entities operatively disposed in wireless communication, e.g., a printer, scanner, desktop or portable computer, portable data assistant, communications satellite, any piece of equipment or location associated with a wirelessly detectable tag (e.g., a kiosk, news stand, store shelf, etc.), and telephone. This can include Wireless Fidelity (Wi-Fi) and BLUETOOTH^{®} wireless technologies. Thus, the communication can be a predefined structure as with a conventional network or simply an ad hoc communication between at least two devices.

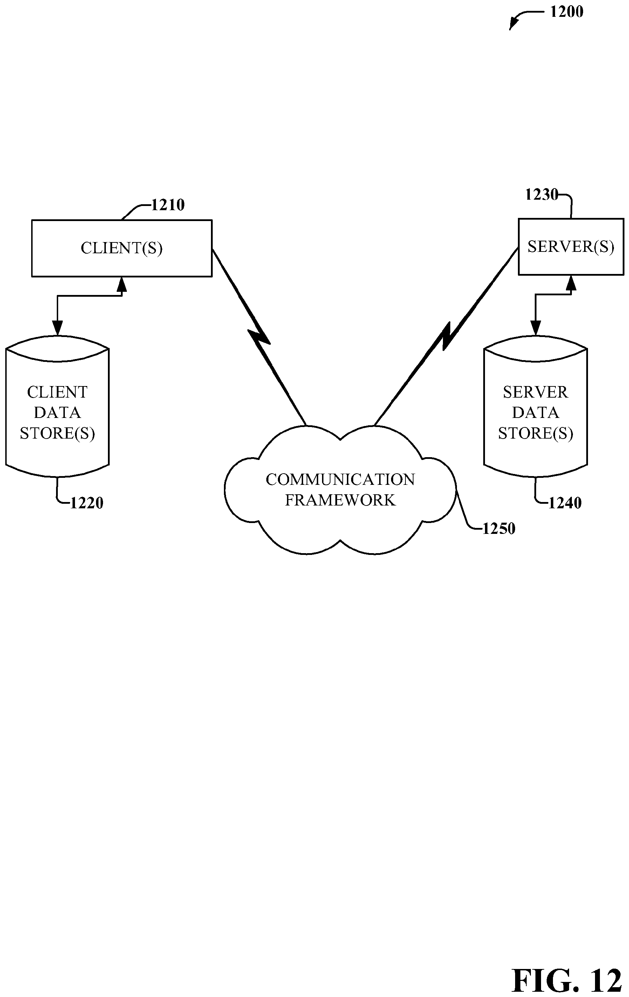

FIG. 12 is a schematic block diagram of a sample computing environment 1200 with which the disclosed subject matter can interact. The sample computing environment 1200 includes one or more client(s) 1210. The client(s) 1210 can be hardware or software (e.g., threads, processes, computing devices). The sample computing environment 1200 also includes one or more server(s) 1230. The server(s) 1230 can also be hardware or software (e.g., threads, processes, computing devices). The servers 1230 can house threads to perform transformations by employing one or more embodiments as described herein, for example. One possible communication between a client 1210 and a server 1230 can be in the form of a data packet adapted to be transmitted between two or more computer processes. The sample computing environment 1200 includes a communication framework 1250 that can be employed to facilitate communications between the client(s) 1210 and the server(s) 1230. The client(s) 1210 are operably connected to one or more client data store(s) 1220 that can be employed to store information local to the client(s) 1210. Similarly, the server(s) 1230 are operably connected to one or more server data store(s) 1240 that can be employed to store information local to the servers 1230.

Various embodiments may be a system, a method, an apparatus or a computer program product at any possible technical detail level of integration. The computer program product can include a computer readable storage medium (or media) having computer readable program instructions thereon for causing a processor to carry out aspects of various embodiments. The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium can also include the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network or a wireless network. The network can comprise copper transmission cables, optical transmission fibers, wireless transmission, routers, firewalls, switches, gateway computers or edge servers. A network adapter card or network interface in each computing/processing device receives computer readable program instructions from the network and forwards the computer readable program instructions for storage in a computer readable storage medium within the respective computing/processing device. Computer readable program instructions for carrying out operations of various embodiments can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, including a local area network (LAN) or a wide area network (WAN), or the connection can be made to an external computer (for example, through the Internet using an Internet Service Provider). In some embodiments, electronic circuitry including, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform various aspects.

Various aspects are described herein with reference to flowchart illustrations or block diagrams of methods, apparatus (systems), and computer program products according to various embodiments. It will be understood that each block of the flowchart illustrations or block diagrams, and combinations of blocks in the flowchart illustrations or block diagrams, can be implemented by computer readable program instructions. These computer readable program instructions can be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart or block diagram block or blocks. These computer readable program instructions can also be stored in a computer readable storage medium that can direct a computer, a programmable data processing apparatus, or other devices to function in a particular manner, such that the computer readable storage medium having instructions stored therein comprises an article of manufacture including instructions which implement aspects of the function/act specified in the flowchart or block diagram block or blocks. The computer readable program instructions can also be loaded onto a computer, other programmable data processing apparatus, or other device to cause a series of operational acts to be performed on the computer, other programmable apparatus or other device to produce a computer implemented process, such that the instructions which execute on the computer, other programmable apparatus, or other device implement the functions/acts specified in the flowchart or block diagram block or blocks.

The flowcharts and block diagrams in the Figures illustrate the architecture, functionality, and operation of possible implementations of systems, methods, and computer program products according to various embodiments. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams or flowchart illustration, and combinations of blocks in the block diagrams or flowchart illustration, can be implemented by special purpose hardware-based systems that perform the specified functions or acts or carry out combinations of special purpose hardware and computer instructions.

While the subject matter has been described above in the general context of computer-executable instructions of a computer program product that runs on a computer or computers, those skilled in the art will recognize that this disclosure also can or can be implemented in combination with other program modules. Generally, program modules include routines, programs, components, data structures, etc. that perform particular tasks or implement particular abstract data types. Moreover, those skilled in the art will appreciate that various aspects can be practiced with other computer system configurations, including single-processor or multiprocessor computer systems, mini-computing devices, mainframe computers, as well as computers, hand-held computing devices (e.g., PDA, phone), microprocessor-based or programmable consumer or industrial electronics, and the like. The illustrated aspects can also be practiced in distributed computing environments in which tasks are performed by remote processing devices that are linked through a communications network. However, some, if not all aspects of this disclosure can be practiced on stand-alone computers. In a distributed computing environment, program modules can be located in both local and remote memory storage devices.

As used in this application, the terms "component," "system," "platform," "interface," and the like, can refer to or can include a computer-related entity or an entity related to an operational machine with one or more specific functionalities. The entities disclosed herein can be either hardware, a combination of hardware and software, software, or software in execution. For example, a component can be, but is not limited to being, a process running on a processor, a processor, an object, an executable, a thread of execution, a program, or a computer. By way of illustration, both an application running on a server and the server can be a component. One or more components can reside within a process or thread of execution and a component can be localized on one computer or distributed between two or more computers. In another example, respective components can execute from various computer readable media having various data structures stored thereon. The components can communicate via local or remote processes such as in accordance with a signal having one or more data packets (e.g., data from one component interacting with another component in a local system, distributed system, or across a network such as the Internet with other systems via the signal). As another example, a component can be an apparatus with specific functionality provided by mechanical parts operated by electric or electronic circuitry, which is operated by a software or firmware application executed by a processor. In such a case, the processor can be internal or external to the apparatus and can execute at least a part of the software or firmware application. As yet another example, a component can be an apparatus that provides specific functionality through electronic components without mechanical parts, wherein the electronic components can include a processor or other means to execute software or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. As used herein, the term "and/or" is intended to have the same meaning as "or." Moreover, articles "a" and "an" as used in the subject specification and annexed drawings should generally be construed to mean "one or more" unless specified otherwise or clear from context to be directed to a singular form. As used herein, the terms "example" or "exemplary" are utilized to mean serving as an example, instance, or illustration. For the avoidance of doubt, the subject matter disclosed herein is not limited by such examples. In addition, any aspect or design described herein as an "example" or "exemplary" is not necessarily to be construed as preferred or advantageous over other aspects or designs, nor is it meant to preclude equivalent exemplary structures and techniques known to those of ordinary skill in the art.

The herein disclosure describes non-limiting examples. For ease of description or explanation, various portions of the herein disclosure utilize the term "each," "every," or "all" when discussing various examples. Such usages of the term "each," "every," or "all" are non-limiting. In other words, when the herein disclosure provides a description that is applied to "each," "every," or "all" of some particular object or component, it should be understood that this is a non-limiting example, and it should be further understood that, in various other examples, it can be the case that such description applies to fewer than "each," "every," or "all" of that particular object or component.

As it is employed in the subject specification, the term "processor" can refer to substantially any computing processing unit or device comprising, but not limited to, single-core processors; single-processors with software multithread execution capability; multi-core processors; multi-core processors with software multithread execution capability; multi-core processors with hardware multithread technology; parallel platforms; and parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, or any combination thereof designed to perform the functions described herein. Further, processors can exploit nano-scale architectures such as, but not limited to, molecular and quantum-dot based transistors, switches and gates, in order to optimize space usage or enhance performance of user equipment. A processor can also be implemented as a combination of computing processing units. In this disclosure, terms such as "store," "storage," "data store," data storage," "database," and substantially any other information storage component relevant to operation and functionality of a component are utilized to refer to "memory components," entities embodied in a "memory," or components comprising a memory. It is to be appreciated that memory or memory components described herein can be either volatile memory or nonvolatile memory, or can include both volatile and nonvolatile memory. By way of illustration, and not limitation, nonvolatile memory can include read only memory (ROM), programmable ROM (PROM), electrically programmable ROM (EPROM), electrically erasable ROM (EEPROM), flash memory, or nonvolatile random access memory (RAM) (e.g., ferroelectric RAM (FeRAM). Volatile memory can include RAM, which can act as external cache memory, for example. By way of illustration and not limitation, RAM is available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM), and Rambus dynamic RAM (RDRAM). Additionally, the disclosed memory components of systems or computer-implemented methods herein are intended to include, without being limited to including, these and any other suitable types of memory.

What has been described above include mere examples of systems and computer-implemented methods. It is, of course, not possible to describe every conceivable combination of components or computer-implemented methods for purposes of describing this disclosure, but many further combinations and permutations of this disclosure are possible. Furthermore, to the extent that the terms "includes," "has," "possesses," and the like are used in the detailed description, claims, appendices and drawings such terms are intended to be inclusive in a manner similar to the term "comprising" as "comprising" is interpreted when employed as a transitional word in a claim.

The descriptions of the various embodiments have been presented for purposes of illustration, but are not intended to be exhaustive or limited to the embodiments disclosed. Many modifications and variations will be apparent without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

## Claims

1. A system, comprising:
a processor (e.g., 108) that executes computer-executable components stored in a non-transitory computer-readable memory (e.g., 110), wherein the computer-executable components comprise:
an access component (e.g., 112) that accesses a plain text service manual (e.g., 202) and a service complaint (e.g., 106) that are associated with a medical imaging scanner (e.g., 104);
a parsing component (e.g., 114) that identifies, via named-entity recognition or natural language processing, a semantic hierarchy (e.g., 302) of the plain text service manual; and
a trace component (e.g., 116) that records, via graphical user interface tracking, how a service technician (e.g., 602) that is troubleshooting the service complaint sequentially navigates through the semantic hierarchy, thereby yielding a troubleshooting trace (e.g., 504) that corresponds to the service complaint.

2. The system of claim 1, wherein the semantic hierarchy is a knowledge graph representation of the plain text service manual, and wherein the troubleshooting trace is a directed path through the knowledge graph representation that indicates a reading sequence employed by the service technician through the plain text service manual to solve the service complaint.

3. The system of claim 1, wherein the access component accesses an operational report (e.g., 902) generated by the medical imaging scanner, wherein the semantic hierarchy is a knowledge graph representation of both the plain text service manual and the operational report, and wherein the troubleshooting trace is a directed path through the knowledge graph representation that indicates a reading sequence employed by the service technician through both the plain text service manual and the operational report to solve the service complaint.

4. The system of claim 1, wherein the graphical user interface tracking comprises click-tracking, scroll-tracking, or eye-movement-tracking.

5. The system of claim 1, wherein the computer-executable components further comprise:
a model component (e.g., 118) that trains a deep learning neural network (e.g., 702) on the service complaint and on the troubleshooting trace, wherein the service complaint is treated as a training input for the deep learning neural network, and wherein the troubleshooting trace is treated as a ground-truth annotation corresponding to the service complaint.

6. The system of claim 5, wherein the model component deploys the deep learning neural network, after training, as a third-party service.

7. The system of claim 1, wherein a node (e.g., one of 402) of the semantic hierarchy is based on a keyword located in the plain text service manual, wherein the keyword is associated with an electronic action performable by the medical imaging scanner, and wherein clicking on or invoking the node or the keyword causes the medical imaging scanner to automatically perform the electronic action.

8. A computer-implemented method, comprising:
accessing, by a device (e.g., via 112) operatively coupled to a processor (e.g., 108), a plain text service manual (e.g., 202) and a service complaint (e.g., 106) that are associated with a medical imaging scanner (e.g., 104);
identifying, by the device (e.g., via 114) and via named-entity recognition or natural language processing, a semantic hierarchy (e.g., 302) of the plain text service manual; and
recording, by the device (e.g., via 116) and via graphical user interface tracking, how a service technician (e.g., 602) that is troubleshooting the service complaint sequentially navigates through the semantic hierarchy, thereby yielding a troubleshooting trace (e.g., 504) that corresponds to the service complaint.

9. The computer-implemented method of claim 8, wherein the semantic hierarchy is a knowledge graph representation of the plain text service manual, and wherein the troubleshooting trace is a directed path through the knowledge graph representation that indicates a reading sequence employed by the service technician through the plain text service manual to solve the service complaint.

10. The computer-implemented method of claim 8, further comprising:
accessing, by the device (e.g., via 112), an operational report (e.g., 902) generated by the medical imaging scanner, wherein the semantic hierarchy is a knowledge graph representation of both the plain text service manual and the operational report, and wherein the troubleshooting trace is a directed path through the knowledge graph representation that indicates a reading sequence employed by the service technician through both the plain text service manual and the operational report to solve the service complaint.

11. The computer-implemented method of claim 8, wherein the graphical user interface tracking comprises click-tracking, scroll-tracking, or eye-movement-tracking.

12. The computer-implemented method of claim 8, further comprising:
training, by the device (e.g., via 118), a deep learning neural network (e.g., 702) on the service complaint and on the troubleshooting trace, wherein the service complaint is treated as a training input for the deep learning neural network, and wherein the troubleshooting trace is treated as a ground-truth annotation corresponding to the service complaint.

13. The computer-implemented method of claim 12, further comprising:
deploying, by the device (e.g., via 118), the deep learning neural network, after training, as a third-party service.

14. The computer-implemented method of claim 8, wherein a node (e.g., one of 402) of the semantic hierarchy is based on a keyword located in the plain text service manual, wherein the keyword is associated with an electronic action performable by the medical imaging scanner, and wherein clicking on or invoking the node or the keyword causes the medical imaging scanner to automatically perform the electronic action.

15. A computer program product for facilitating electronic acquisition of troubleshooting knowledge, the computer program product comprising a non-transitory computer-readable memory (e.g., 110) having program instructions embodied therewith, the program instructions executable by a processor (e.g., 108) to cause the processor to:
access one or more plain text documents (e.g., 202 or 902) associated with a machine (e.g., 104);
identify, via named-entity recognition or natural language processing, a semantic hierarchy (e.g., 302) of the one or more plain text documents;
record, via graphical user interface tracking, how a service technician (e.g., 602) that is troubleshooting a service complaint (e.g., 106) associated with the machine sequentially navigates through the semantic hierarchy, thereby yielding a troubleshooting trace (e.g., 504) that corresponds to the service complaint; and
train a deep learning neural network (e.g., 702) on the service complaint and on the troubleshooting trace, with the service complaint being a training input, and with the troubleshooting trace being a ground-truth annotation.
